# EUROPEAN PATENT APPLICATION

(11) **EP 1 867 343 A2**
(43) Date of publication of application: **19.12.2007**
(21) Application number: 07117850.3
(22) Date of filing: 23.05.2003
(51) Int. Cl.: A61K 47/48

(54) **Novel compounds**

(30) Priority: 23.05.2002 GB 0211949
(62) Divisional of application: 03727695.3
(71) Applicant: GLAXO GROUP LIMITED, Greenford, Middlesex UB6 0NN (GB)
(72) Inventor: Craig, Andrew Simon, Tonbridge, Kent TN11 9AN (GB); Crowe, David Malcolm, Tonbridge, Kent TN11 9AN (GB); Millan, Michael John, Tonbridge, Kent TN11 9AN (GB)
(74) Representative: Rutter, Keith

(57) **Abstract**

A host-guest complex, wherein the host is a cyclodextrin or a mixture of cyclodextrins (the Cyclodextrin) and the guest is 5-[4-[2-(N-methyl-N-(2-pyridyl)amino)ethoxy]benzyl]thiazolidine-2,4-dione, (Compound (I)) or a pharmaceutically acceptable derivative thereof or a mixture thereof; a process for the preparation of said complex, a pharmaceutical composition comprising such complex and the use of such complex in medicine.

## Description

This invention relates to novel compounds and compositions, to a process to prepare such compounds and compositions and to the use of such compounds and compositions in medicine.

European Patent Application, Publication Number 0,306,228 relates to certain thiazolidinedione derivatives disclosed as having hypoglycaemic and hypolipidaemic activity including 5-[4-[2-(N-methyl-N-(2-pyridyl)amino)ethoxy]benzyl]thiazolidine-2,4-dione, (hereinafter also referred to as "Compound (I)").

International Patent Application, Publication Number WO94/05659 discloses certain thiazolidinedione derivatives having hypoglycaemic and hypolipidaemic activity including 5-[4-[2-(N-methyl-N-(2-pyridyl)amino)ethoxy]benzyl]thiazolidine-2,4-dione, maleic acid salt.

International Patent Applications, Publication Numbers WO 00/63206, WO 00/94343 and WO 00/94343 also disclose certain salts of Compound (I).

Surprisingly, it has now been found that Compound (I) and certain derivatives of Compound (I) form crystalline and non-crystalline inclusion complexes and compositions with cyclodextrins. These novel inclusion complexes and compositions are indicated to exhibit enhanced solubility characteristics and physical properties and hence provide potential bulk handling and formulation advantages

Accordingly, the invention provides a host-guest complex, wherein the host is a cyclodextrin or a mixture of cyclodextrins (hereinafter also referred to as "the Cyclodextrin") and the guest is Compound (I) or a pharmaceutically acceptable derivative thereof or a mixture thereof.

In the accompanying drawings:
Figure 1 is an X-Ray Diffraction Pattern of the product of Example 1;
Figure 2 is an X-Ray Diffraction Pattern of the product of Example 2;
Figure 3 is an X-Ray Diffraction Pattern of the product of Example 3;
Figure 4 is an X-Ray Diffraction Pattern of the product of Example 4;
Figure 5 is an X-Ray Diffraction Pattern of the product of Example 5;
Figure 6 is an X-Ray Diffraction Pattern of the product of Example 6;
Figure 7 is an X-Ray Diffraction Pattern of the product of Example 7;
Figure 8 is an X-Ray Diffraction Pattern of the product of Example 8;
Figure 9 is an X-Ray Diffraction Pattern of the product of Example 10;
Figure 10 is an X-Ray Diffraction Pattern of the product of Example 11;
Figure 11 is an X-Ray Diffraction Pattern of the product of Example 12;
Figure 12 is an X-Ray Diffraction Pattern of the product of Example 13;
Figure 13 is an X-Ray Diffraction pattern of the product of Example 14;
Figure 14 is an X-Ray Diffraction Pattern of the product of Example 15;
Figure 15 is an X-Ray Diffraction Pattern of the product of Example 17;
Figure 16 is an X-Ray Diffraction Pattern of the product of Example 19;
Figure 17 is an X-Ray Diffraction Pattern of the product of Example 20;
Figure 18 is an X-Ray Diffraction Pattern of the product of Example 21;
Figure 19 is an X-Ray Diffraction Pattern of the product of Example 22;
Figure 20 is an X-Ray Diffraction Pattern of the product of Example 25;
Figure 21 is an X-Ray Diffraction Pattern of the product of Example 29;
Figure 22 is an X-Ray Diffraction Pattern of the product of Example 30;
Figure 23 is an X-Ray Diffraction Pattern of the product of Example 32; and
Figure 24 is a Plot of Time vs Absorbance for Examples 23, 24 and 27 and the maleate salt of Compound (I)

Suitable complexes are crystalline complexes.

Suitable complexes also include certain non-crystalline complexes, especially when the Cyclodextrin is (itself) non-crystalline for example a hydroxypropylated cyclodextrin.

Suitably, the complexes are stoichiometric or substantially stoichiometric, preferably having a stoichiometry of within the range of between about 3: 1 to about 1:1 the Cyclodextrin to Compound (I), for example 2:1 the Cyclodextrin to Compound (I) or 1: 1 the Cyclodextrin to Compound (I).

Suitably, for an approximate 1:1 complex the ratio of Cyclodextrin to Compound (I) is in the range of about 0.7:1 and 1.3: 1 and for an approximate 2:1 complex in the range of about 1.7:1 and 2.5:1.

For the avoidance of doubt, non-stoichiometric complexes also form part of the invention.

The Cyclodextrin includes those selected from the group consisting of α, β and γ cyclodextrins, methylated cyclodextrins, hydroxypropyl cyclodextrins e.g. hydroxypropyl-β-cyclodextrin (HPBCD) and hydroxypropyl-γ-cyclodextrin, hydroxyethyl cyclodextrins e.g. hydroxyethyl- β-cyclodextrin (HEBCD), branched cyclodextrins in which one or two glucoses or maltoses are attached to the cyclodextrin ring, ethyl- and ethyl-carboxymethyl cyclodextrins, dihydroxypropyl cyclodextrins, and sulfoalkyl ether cyclodextrins. The degree of substitution is not considered to be critical and the cyclodextrin used can have essentially any degree of substitution (per entire cyclodextrin molecule). The use of β- or γ cyclodextrins is preferred. Mixtures of cyclodextrins, as well as single species may be used in the present invention. The Cyclodextrin may be in anhydrous or hydrous form.

A suitable Cyclodextrin is a α cyclodextrin. A suitable Cyclodextrin is a β cyclodextrin. A suitable Cyclodextrin is a γ cyclodextrin. A suitable Cyclodextrin is a methylated cyclodextrin. A suitable Cyclodextrin is a hydroxypropyl cyclodextrin.

One Cyclodextrin is a hydroxypropyl-β-cyclodextrin (HPBCD). A further Cyclodextrin is a hydroxypropyl-γ-cyclodextrin.

Suitable, pharmaceutically acceptable derivatives of Compound (I) include pharmaceutically acceptable salts and solvates thereof.

Suitable, pharmaceutically acceptable salts include acid salts or base salts. Suitable acid salts include those provided by pharmaceutically acceptable acids, especially mineral acids for example hydrogen chloride, or organic acids such as carboxylic acids e.g. maleic acid, or sulphonic acids e.g. methanesulfonic acid. Examples of acid salts include 5-[4-[2-(N-methyl-N-(2-pyridyl)amino)ethoxy]benzyl]thiazolidine-2,4-dione, hydrochloride salt and 5-[4-[2-(N-methyl-N-(2-pyridyl)amino)ethoxy]benzyl]thiazolidine-2,4-dione, maleate salt. Suitable base salts include those provided by pharmaceutically acceptable bases e.g. with group IA/IIA metal salts e.g. sodium, potassium or organic bases such as choline. An example of a base salt is 5-[4-[2-(N-methyl-N-(2-pyridyl)amino)ethoxy]benzyl]thiazolidine-2,4-dione, potassium salt.

A suitable solvate is a hydrate.

Suitable derivatives, including salts, also include each of those disclosed in the above mentioned patent publications.

Suitably, the guest is Compound (I), that is 5-[4-[2-(N-methyl-N-(2-pyridyl)amino)ethoxy]benzyl]thiazolidine-2,4-dione.

Suitably, the guest is an acid salt of Compound (I). Suitable acid salts of Compound (I) include maleate and hydrochloride salts

Suitably, the guest is a base salt of Compound (I). Suitable base salts of Compound (I) include sodium and potassium salts.

The present invention also provides a process for the preparation of a host-guest complex, wherein the host is a cyclodextrin or a mixture of cyclodextrins (hereinafter also referred to as "the Cyclodextrin") and the guest is Compound (I) or a pharmaceutically acceptable derivative thereof or a mixture thereof, which process comprises contacting the Cyclodextrin and Compound (I) or a pharmaceutically acceptable derivative thereof or a mixture thereof in an appropriate liquid (solvent); and thereafter, if required, isolating the host-guest complex from the reaction mixture.

Suitable contacting methods include dissolving the required amount of the Cyclodextrin and Compound (I), or a pharmaceutically acceptable derivative thereof, in a liquid or admixing liquid solutions or dispersions of the Cyclodextrin and Compound (I), or a pharmaceutically acceptable derivative thereof, so as to form the required host-guest complex.

The liquids chosen for the process will depend upon the particular contacting method chosen but generally they are selected from water or aqueous solvent mixtures, including water admixed with an alcohol, such as propan-1-ol, propan-2-ol and ethanol; an organic acid such as acetic acid; a nitrile such as acetonitrile; a ketone such as acetone; or an ether such as tetrahydrofuran.

Where a mixture of water and non-aqueous solvent(s) is employed, preferably the mixture contains a substantial amount of water, for example 20% w/w or more water.

The process may further comprise isolating the host-guest inclusion complex. Isolation may be achieved by any one or more of the methods of precipitation, concentration and/or drying (which may result in crystalline or non-crystalline product). Precipitation may be achieved by cooling the reaction system and/or concentrating the reaction system or by the addition of miscible or partly miscible solvent ("anti-solvent"), such as diethyl ether. In some cases precipitation may be achieved by either acidification, basification or neutralisation of the reaction e.g. neutralisation of a solution comprising compound (I) hydrochloride salt and cyclodextrin by addition of a base such as potassium carbonate. Concentration and drying may be achieved by methods such as freeze drying, vacuum evaporation, vacuum oven drying, spray drying, or air or inert atmosphere drying of the reaction mixture or any combination thereof.

In one particular form of the process, the formation of the host-guest inclusion complex is achieved by contacting Cyclodextrin and Compound (I), or a pharmaceutically acceptable derivative thereof, and a liquid, preferably water, to form a paste, which is then kneaded. The host-guest inclusion complex may subsequently be isolated by drying the paste typically by evaporation, vacuum oven drying, or air or inert atmosphere drying. In an alternative aspect, an inclusion complex is formed by adding a Cyclodextrin to a solution of Compound (I), or a pharmaceutically acceptable derivative thereof, in water or an organic solvent or a mixture thereof. The resulting inclusion complex is then isolated by addition of an anti-solvent or by cooling, evaporating or drying the reaction mixture. Alternatively, the inclusion complex may be isolated by neutralising the reaction mixture using, for example, potassium carbonate..

In a further embodiment of the process, the host-guest inclusion complex is formed by adding a solution of Compound (I), or a pharmaceutically acceptable derivative thereof to an aqueous solution of a Cyclodextrin.

In a more particular form the process comprises:
(a) the Cyclodextrin and Compound (I) and/or a pharmaceutically acceptable derivative thereof are admixed with water, preferably with the minimum amount of water, and generally in the range of from 20-80% w/w water with respect to the total weight of the reactants, wherein the admixing/kneading process is continued until the complex formation is complete, generally over a time period of from 10 to 60 minutes, for example 30 minutes; or
(b) neutralising a solution or dispersion of the Cyclodextrin and a derivative (salt) of Compound (I), in an aqueous liquid such as a water or water admixed with a lower alcohol, tetrahydrofuran, acetonitrile or acetone wherein the neutralisation is effected by addition of an appropriate neutralising reagent suitably being a base, such as aqueous potassium carbonate solution or an acid such as hydrochloric acid or acetic acid as appropriate, so as to precipitate the required host-guest composition; or
(c) admixing a solution of Compound (I)), and/or a pharmaceutically acceptable derivative thereof, in a first solvent, such as an alcohol, tetrahydrofuran, acetonitrile, acetic acid or acetone with a solution of a Cyclodextrin in a second solvent, such as water or mixtures of water with lower alcohols, tetrahydrofuran, acetonitrile, acetic acid or acetone wherein the first and second solvent are chosen such that the required host-guest composition precipitates from the solvent mixture, preferably the solution of the Cyclodextrin, especially when β-cyclodextrin, is heated above ambient temperature to facilitate solubilisation, for example a temperature within the range of from 35 - 100°C or the reflux temperature of the solvent, preferably 40-80°C and the solvent mixture is cooled to facilitate precipitation, for example to a temperature within the range of from 0-30°C ; or
(d) admixing a solution of the Cyclodextrin and Compound (I), and/or a pharmaceutically acceptable derivative thereof, in an aqueous liquid, such as a water or water admixed with a lower alcohol, tetrahydrofuran or acetonitrile or acetone with an appropriate anti-solvent so that the required host-guest composition precipitates from the solvent mixture; or
(e) preparing a solution of the host-guest composition by mixing Cyclodextrin and Compound (I), and/or a pharmaceutically acceptable derivative thereof, in an appropriate solvent, such as a water or water admixed with a lower alcohol, tetrahydrofuran or acetonitrile or acetone and thereafter removing the solvent, suitably by evaporation induced by heating and /or vacuum or by freeze drying; or
(f) generating a dispersion of Cyclodextrin and a Compound (I), and/or a pharmaceutically acceptable derivative thereof, in a liquid, such as water, a lower alcohol, tetrahydrofuran, acetic acid or a mixture thereof, and stirring until complex formation is complete; and thereafter as necessary isolating the required host-guest composition, for example by filtration.

Process a) above is conveniently carried out on a large/commercial scale in a kneading type mixing apparatus, such as an extruder or σ blade mixer.

Where the product is isolated by crystallisation careful control of precipitation temperature and seeding may be used to improve the reproducibility of the product form.

Crystallisation can also be initiated by seeding with crystals of the required product, but this is not essential.

Compound (I) is prepared according to known procedures, such as those disclosed in EP 0,306,228 and WO94/05659. The disclosures of EP 0,306,228 and WO94/05659 are incorporated herein by reference.

The term "host-guest complex" has a well known meaning in the art and in the present application relates to compounds wherein the Cyclodextrin acts as the host and Compound (I), or a pharmaceutically acceptable derivative thereof or a mixture thereof, is the guest.

Suitably, Compound (I), or a pharmaceutically acceptable derivative thereof, is associated with a molecular cavity provided by the Cyclodextrin, typically Compound (I), or a pharmaceutically acceptable derivative thereof, is located wholly or partially within the molecular cavity provided by the Cyclodextrin.

When used herein the term 'prophylaxis of conditions associated with diabetes mellitus' includes the treatment of conditions such as insulin resistance, impaired glucose tolerance, hyperinsulinaemia and gestational diabetes.

Diabetes mellitus preferably means Type II diabetes mellitus.

Conditions associated with diabetes include hyperglycaemia and insulin resistance and obesity. Further conditions associated with diabetes include hypertension, cardiovascular disease, especially atherosclerosis, certain eating disorders, in particular the regulation of appetite and food intake in subjects suffering from disorders associated with under-eating, such as anorexia nervosa, and disorders associated with over-eating, such as obesity and anorexia bulimia. Additional conditions associated with diabetes include polycystic ovarian syndrome and steroid induced insulin resistance.

The complications of conditions associated with diabetes mellitus encompassed herein includes renal disease, especially renal disease associated with the development of Type II diabetes including diabetic nephropathy, glomerulonephritis, glomerular sclerosis, nephrotic syndrome, hypertensive nephrosclerosis and end stage renal disease.

As mentioned above the complex of the invention has useful therapeutic properties: The present invention accordingly provides a host-guest complex, wherein the host is a Cyclodextrin and the guest is Compound (I) or a pharmaceutically acceptable derivative thereof or a mixture thereof, for use as an active therapeutic substance.

More particularly, the present invention provides a host-guest complex, wherein the host is a Cyclodextrin and the guest is Compound (I) or a pharmaceutically acceptable derivative thereof or a mixture thereof, for use in the treatment and/or prophylaxis of diabetes mellitus, conditions associated with diabetes mellitus and certain complications thereof.

The present invention provides a host-guest complex, wherein the host is a Cyclodextrin and the guest is Compound (I) or a pharmaceutically acceptable derivative thereof or a mixture thereof, for use in the treatment and/or prophylaxis of Alzheimer's disease.

In addition, the present invention provides a host-guest complex, wherein the host is a Cyclodextrin and the guest is Compound (I) or a pharmaceutically acceptable derivative thereof or a mixture thereof, for use in the treatment and/or prophylaxis of psoriasis.

The host-guest complex may be administered per se or, preferably, as a pharmaceutical composition also comprising a pharmaceutically acceptable carrier. Suitable methods for formulating the host-guest complex are generally those disclosed for Compound (I) in the publications mentioned herein.

Accordingly, the present invention also provides a pharmaceutical composition comprising a host-guest complex, wherein the host is a Cyclodextrin and the guest is Compound (I) or a pharmaceutically acceptable derivative thereof or a mixture thereof, and a pharmaceutically acceptable carrier therefor

The host-guest complex is normally administered in unit dosage form.

The active compound may be administered by any suitable route but usually by the oral or parenteral routes. For such use, the complex will normally be employed in the form of a pharmaceutical composition in association with a pharmaceutical carrier, diluent and/or excipient, although the exact form of the composition will naturally depend on the mode of administration.

Compositions are prepared by admixture and are suitably adapted for oral, parenteral or topical administration, and as such may be in the form of tablets, capsules, oral liquid preparations, powders, granules, lozenges, pastilles, reconstitutable powders, injectable and infusable solutions or suspensions, suppositories and transdermal devices. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

Solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the active compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the active compound is suspended in the vehicle instead of being dissolved and sterilised by exposure to ethylene oxide before suspending in the sterile vehicle. Advantageously, a surfactant or wetting agent is included in the composition to facilitate uniform distribution of the active compound.

As is common practice, the compositions will usually be accompanied by written or printed directions for use in the medical treatment concerned.

As used herein the term 'pharmaceutically acceptable' embraces compounds, compositions and ingredients for both human and veterinary use: for example the term 'pharmaceutically acceptable salt' embraces a veterinarily acceptable salt.

The present invention further provides a method for the treatment and/or prophylaxis of diabetes mellitus, conditions associated with diabetes mellitus and certain complications thereof, in a human or non-human mammal which comprises administering an effective, non-toxic, amount of a host-guest complex, wherein the host is a Cyclodextrin and the guest is Compound (I) or a pharmaceutically acceptable derivative thereof or a mixture, thereof to a human or non-human mammal in need thereof.

In addition, the present invention further provides a method for the treatment and/or prophylaxis of Alzheimer's disease, in a human or non-human mammal which comprises administering an effective, non-toxic, amount of a host-guest complex, wherein the host is a Cyclodextrin and the guest is Compound (I) or a pharmaceutically acceptable derivative thereof or a mixture, thereof to a human or non-human mammal in need thereof.

Further, the present invention further provides a method for the treatment and/or prophylaxis of psoriasis, in a human or non-human mammal which comprises administering an effective, non-toxic, amount of a host-guest complex, wherein the host is a Cyclodextrin and the guest is Compound (I) or a pharmaceutically acceptable derivative thereof or a mixture, thereof to a human or non-human mammal in need thereof.

Conveniently, the active ingredient may be administered as a pharmaceutical composition hereinbefore defined, and this forms a particular aspect of the present invention.

In a further aspect the present invention provides the use of a host-guest complex, wherein the host is a Cyclodextrin and the guest is Compound (I) or a pharmaceutically acceptable derivative thereof or a mixture thereof, for the manufacture of a medicament for the treatment and/or prophylaxis of diabetes mellitus, conditions associated with diabetes mellitus and certain complications thereof.

The present invention provides the use of a host-guest complex, wherein the host is a Cyclodextrin and the guest is Compound (I) or a pharmaceutically acceptable derivative thereof or a mixture thereof, for the manufacture of a medicament for the treatment and/or prophylaxis of Alzheimer's disease or psoriasis..

In the treatment mentioned herein including the treatment and/or prophylaxis of diabetes mellitus, conditions associated with diabetes mellitus and certain complications thereof, Alzheimer's disease and psoriasis the host-guest complex, may be taken in amounts so as to provide Compound (I) in suitable doses, such as those disclosed in EP 0,306,228, WO94/05659 or WO98/55122.

The unit dose compositions of the invention comprise a host-guest complex, wherein the host is a Cyclodextrin and the guest is Compound (I) or a pharmaceutically acceptable derivative thereof or a mixture thereof, in an amount providing up to 12mg, including 1-12mg such as 2-12mg of Compound (I), especially 2-4mg, 4-8mg or 8-12mg of Compound (I), for example 1, 2, 3, 4, 5, 6, 7, 8, 9, 10, 11 or 12mg of Compound (I). Thus in particular there is provided a pharmaceutical composition comprising the host-guest complex, wherein the host is a Cyclodextrin and the guest is Compound (I) or a pharmaceutically acceptable derivative thereof or a mixture thereof and a pharmaceutically acceptable carrier therefor, wherein Compound (I) is present in an amount providing 1, 2, 4, 8, 4 to 8 or 8 to 12mg of Compound (I); such as 1mg of Compound (I); such as 2mg of Compound (I); such as 4mg of Compound (I); such as 8mg of Compound (I); such as 12mg of Compound (I).

The invention also provides a pharmaceutical composition comprising a host-guest complex, wherein the host is a Cyclodextrin and the guest is Compound (I) or a pharmaceutically acceptable derivative thereof or a mixture thereof in combination with one or more other pharmaceutically acceptable agents, such as an anti-diabetic agent and optionally a pharmaceutically acceptable carrier therefor.

The invention also provides a method for the treatment and/or prophylaxis of diabetes mellitus, conditions associated with diabetes mellitus and certain complications thereof, in a human or non-human mammal which comprises administering an effective, non-toxic, amount of the host-guest complex, wherein the host is a Cyclodextrin and the guest is Compound (I) or a pharmaceutically acceptable derivative thereof or a mixture thereof, in combination with one or more other anti-diabetic agents.

In a further aspect the present invention provides the use of a host-guest complex, wherein the host is a Cyclodextrin and the guest is Compound (I) or a pharmaceutically acceptable derivative thereof or a mixture thereof in combination with one or more other anti-diabetic agents, for the manufacture of a medicament for the treatment and/or prophylaxis of diabetes mellitus, conditions associated with diabetes mellitus and certain complications thereof.

In the above mentioned treatments the administration of the host-guest complex and the other pharmaceutically acceptable agent or agents, such as the anti-diabetic agent, includes co-administration or sequential administration of the active agents.

Suitably in the above mentioned combination compositions, including unit doses, or treatments the host-guest complex is present in an amount providing up to 12mg, including 1-12mg, such as 2-12mg of Compound (I), especially 2-4mg, 4-8mg or 8-12mg of Compound (I), for example 1, 2, 3, 4, 5, 6, 7, 8, 9, 10, 11, or 12mg of Compound (I) or 4 to 8 or 8 to 12 mg of Compound (I). Thus for example in the above mentioned compositions, including unit doses, or treatments the host-guest complex is present in an amount providing 1mg of Compound (I); the host-guest complex is present in an amount providing 2mg of Compound (I); the host-guest complex is present in an amount providing 3mg of Compound (I); the host-guest complex is present in an amount providing 4mg of Compound (I); or the host-guest complex is present in an amount providing 8mg of Compound (I).

The pharmaceutically acceptable agents, such as the antidiabetic agent, are those disclosed in in standard reference texts, for example the British and US Pharmacopoeias, Remington's Pharmaceutical Sciences (Mack Publishing Co.) and Martindale The Complete Drug Reference (London, The Pharmaceutical Press),

In particular, when the other pharmaceutically acceptable agent ia an antidiabetic agent suitable agents are selected from biguanides, sulphonylureas and alpha glucosidase inhibitors. The other antidiabetic agent is suitably a biguanide. The other antidiabetic agent is suitably a sulphonylurea. The other antidiabetic agent is suitably a alpha glucosidase inhibitor. Suitable antidiabetic agents are those disclosed in WO98/57649, WO98/57634, WO98/57635, WO98/57636, WO99/03477, WO99/03476.

In the accompanying drawings:
The contents of the above mentioned publications are incorporated herein by reference as if set forth in full herein.

No adverse toxicological effects are indicated in the above mentioned treatments for the compounds of the invention.

The following Examples illustrate the invention but do not limit it in any way.

### Examples

### Example 1: Preparation of 5-[4-[2-(N-methyl-N-(2-pyridyl)amino)ethoxy]benzyl] thiazolidine-2,4-dione β-cyclodextrin complex

β-Cyclodextrin (5.0 g) and water (1.5 mL) were ground to a paste in a mortar. 5-[4-[2-(N-Methyl-N-(2-pyridyl)amino)ethoxy]benzyl]thiazolidine-2,4-dione (1.57 g) was then added to the paste along with water (1.5 mL). The mixture was ground for a further 10 minutes and then dried for 1 hour at 21°C and for 2 hours at 50°C under vacuum to afford a 1:1 5-[4-[2-(N-methyl-N-(2-pyridyl)amino)ethoxy]benzyl]thiazolidine-2,4-dione β-cyclodextrin complex.

The solid was recrystallised from a mixture of ethanol (25 ml) and water (20 ml), and then dried under vacuum for 19 hours at 50°C to afford a 1:1 5-[4-[2-(N-methyl-N-(2-pyridyl)amino)ethoxy]benzyl]thiazolidine-2,4-dione β-cyclodextrin complex.

¹H-NMR (d⁶-DMSO): consistent with approximately 1:1 5-[4-[2-(N-methyl-N-(2-pyridyl)amino)ethoxy] benzyl]thiazolidine-2,4-dione β-cyclodextrin complex.

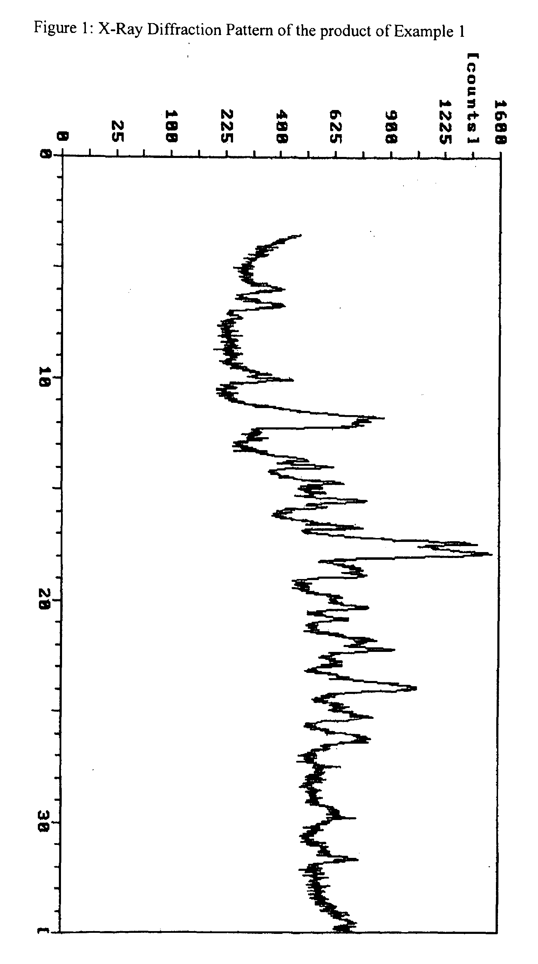

XRPD: See Figure 1.

### Example 2: Preparation of a of 5-[4-[2-(N-methyl-N-(2-pyridyl)amino)ethoxy] benzyl]thiazolidine-2,4-dione β-cyclodextrin complex

A stirred suspension of 5-[4-[2-(N-methyl-N-(2-pyridyl)amino)ethoxy]benzyl]thiazolidine-2,4-dione hydrochloride anhydrate (1.74 g) in water (50 mL) with 2 drops of hydrochloric acid added was heated to 75°C whereupon a clear solution was formed. β-Cyclodextrin (5.0 g) was then added, producing a clear solution within 2 minutes. The solution was cooled to 21°C over approximately 10 minutes before a solution of potassium carbonate (0.85 g) in water (5 mL) was added, giving a white precipitate. The solid was collected by filtration, washed with water (10 mL) and dried at 50°C under vacuum for 4 hours to afford 1:1 5-[4-[2-(N-methyl-N-(2-pyridyl)amino)ethoxy]benzyl]thiazolidine-2,4-dione β-cyclodextrin complex.

¹H-NMR (d⁶-DMSO): consistent with approximately 1:1 5-[4-[2-(N-methyl-N-(2-pyridyl)amino)ethoxy]benzyl]thiazolidine-2,4-dione β-cyclodextrin complex.

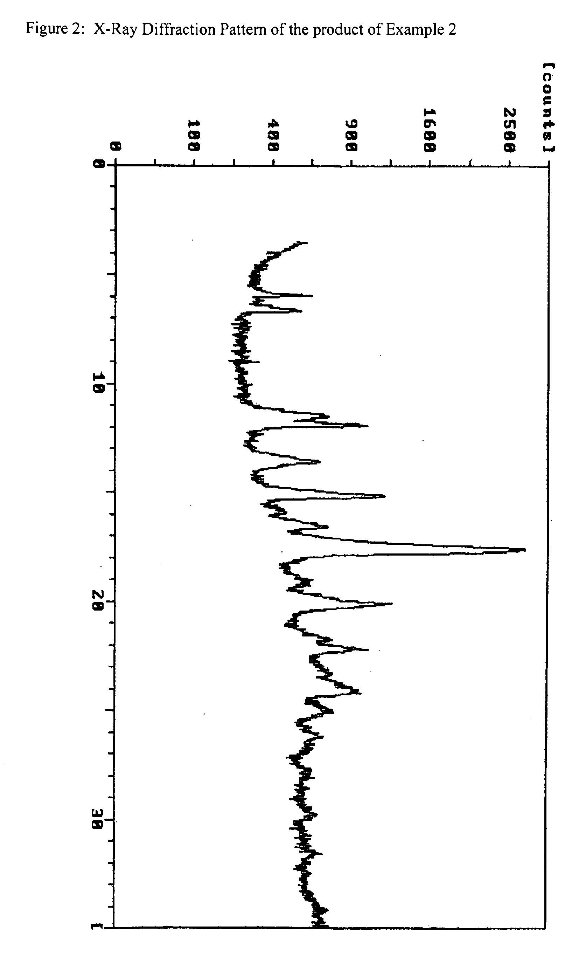

XRPD: See Figure 2.

### Example 3: Preparation of a 5-[4-[2-(N-methyl-N-(2-pyridyl)amino)ethoxy]benzyl] thiazolidine-2,4-dione β-cyclodextrin complex

The product of Example 2 was recrystallised from ethanol (25 ml) and water (20 ml) and dried under vacuum for 16 hours to afford a 1:1 5-[4-[2-(N-methyl-N-(2-pyridyl)amino) ethoxy]benzyl]thiazolidine-2,4-dione β-cyclodextrin complex.

¹H-NMR (d⁶-DMSO): consistent with approximately 1:1 5-[4-[2-(N-methyl-N-(2-pyridyl)amino)ethoxy]benzyl]thiazolidine-2,4-dione β-cyclodextrin complex.

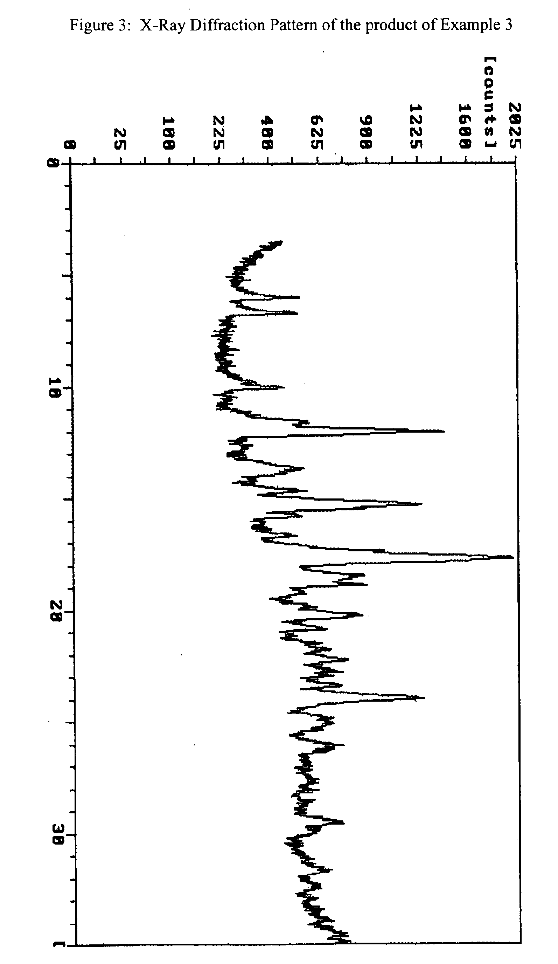

XRPD: See Figure 3. XRPD is consistent with that of the product of Example 1 (Figure 1).

### Example 4: Preparation of a 5-[4-[2-(N-methyl-N-(2-pyridyl)amino)ethoxy]benzyl] thiazolidine-2,4-dione β-cyclodextrin complex

A stirred mixture of 5-[4-[2-(N-methyl-N-(2-pyridyl)amino)ethoxy]benzyl]thiazolidine-2,4-dione (0.79 g) and β-cyclodextrin (5.0 g) in water (40 mL) and ethanol (40 mL) was heated to reflux and maintained at that temperature until a clear solution was formed. The solution was cooled to 21°C over approximately 1 hour. The mixture was maintained at 21 °C until crystallisation was observed to be complete before the solid was recovered by filtration and washed with ethanol. The solid was dried at 50°C under vacuum to afford 1:2 5-[4-[2-(N-methyl-N-(2-pyridyl)amino)ethoxy]benzyl]thiazolidine-2,4-dione β-cyclodextrin complex (4.61 g).

¹H-NMR (d⁶-DMSO): consistent with approximately 1:2 5-[4-[2-(N-methyl-N-(2-pyridyl)amino)ethoxy]benzyl]thiazolidine-2,4-dione β-cyclodextrin complex.

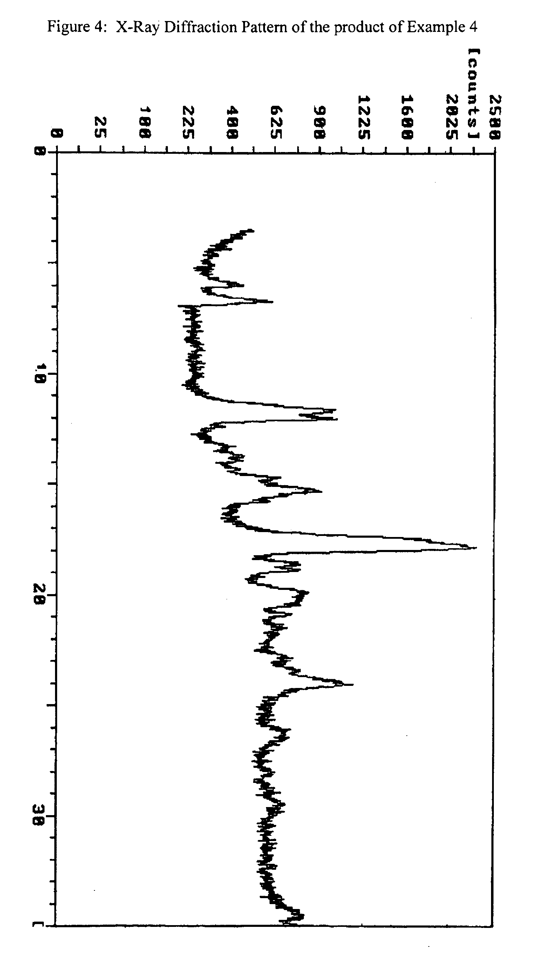

XRPD: See Figure 4.

### Example 5: Preparation of a 5-[4-[2-(N-methyl-N-(2-pyridyl)amino)ethoxy]benzyl] thiazolidine-2,4-dione β-cyclodextrin complex

A 1.0 g sample of the product of Example 4 was recrystallised from a mixture of ethanol (25 ml) and water (5 ml), where the initial solution formed was concentrated to approximately half volume, to afford 1:2 5-[4-[2-(N-methyl-N-(2-pyridyl)amino)ethoxy]benzyl]thiazolidine-2,4-dione β-cyclodextrin complex (0.9 g).

¹H-NMR (d⁶-DMSO): consistent with approximately 1:2 5-[4-[2-(N-methyl-N-(2-pyridyl)amino)ethoxy]benzyl]thiazolidine-2,4-dione β-cyclodextrin complex.

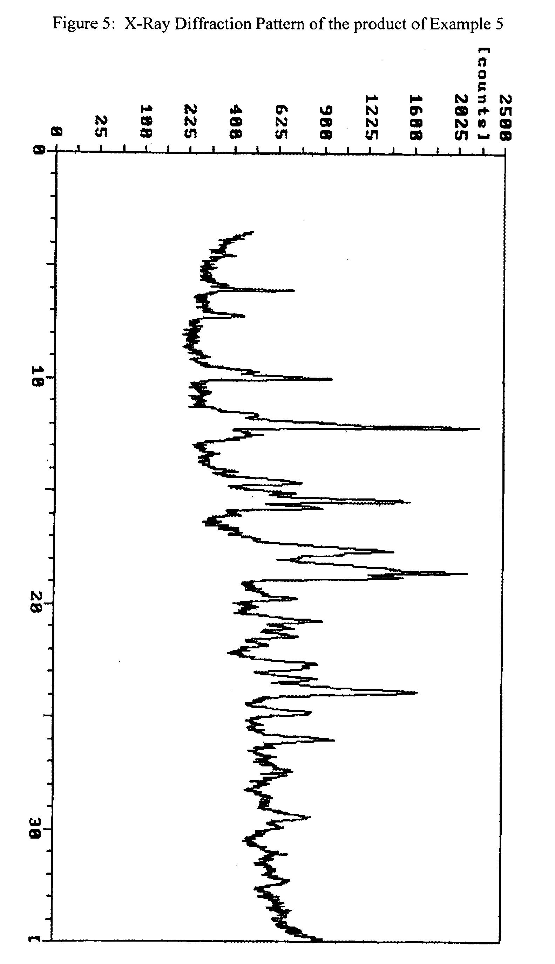

XRPD: See Figure 5.

### Example 6: Preparation of a 5-[4-[2-(N-methyl-N-(2-pyridyl)amino)ethoxy]benzyl] thiazolidine-2,4-dione γ-cyclodextrin complex

5-[4-[2-(N-Methyl-N-(2-pyridyl)amino)ethoxy]benzyl]thiazolidine-2,4-dione hydrochloride dihydrate (0.6g) was added in portions to a stirred solution of γ-cyclodextrin hydrate (4g) in water at 21 °C to give a clear solution. Aqueous potassium carbonate (2 ml, 50%w/v) was added and a white solid was precipitated immediately. The suspension was stirred for a further 1 hour then the product was filtered and dried *in vacuo* to afford an approximately 1:2 5-[4-[2-(N-methyl-N-(2-pyridyl)amino)ethoxy]benzyl]thiazolidine-2,4-dione γ-cyclodextrin complex.

¹H-NMR consistent with the formation of an approximately 1:2 5-[4-[2-(N-methyl-N-(2-pyridyl)amino)ethoxy]benzyl]thiazolidine-2,4-dione- γ-cyclodextrin complex.

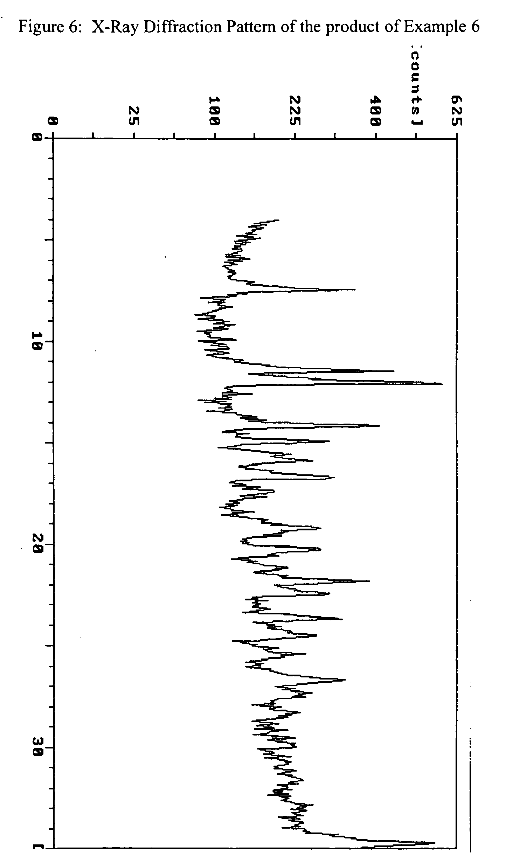

XRPD: See Figure 6.

### Example 7: Preparation of a 5-[4-[2-(N-methyl-N-(2-pyridyl)amino)ethoxy]benzyl] thiazolidine-2,4-dione γ-cyclodextrin complex

A 1.5g sample of the product from Example 6 was recrystallised from water (15 ml) to afford an approximately 1:1 5-[4-[2-(N-methyl-N-(2-pyridyl)amino)ethoxy]benzyl]thiazolidine-2,4-dione γ-cyclodextrin complex.

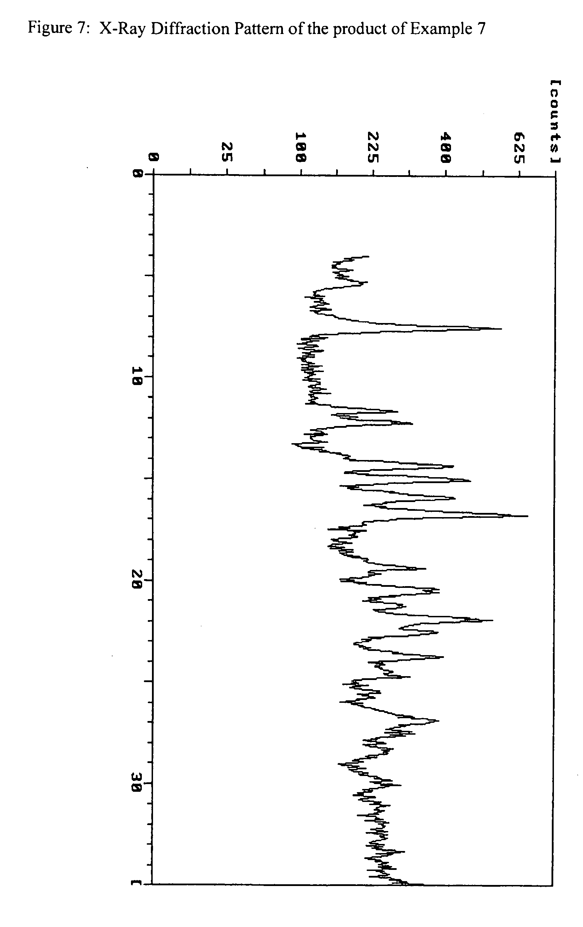

XRPD: See Figure 7.

### Example 8: Preparation of a 5-[4-[2-(N-methyl-N-(2-pyridyl)amino)ethoxy]benzyl] thiazolidine-2,4-dione γ-cyclodextrin complex

A stirred mixture of 5-[4-[2-(N-methyl-N-(2-pyridyl)amino)ethoxy]benzyl]thiazolidine-2,4-dione (1.38 g) and γ-cyclodextrin (5.0 g) in ethanol (45 mL) and water (5 mL) was heated to reflux. After 15 minutes a further portion of water (20 mL) was added, giving a clear solution. The solution was maintained at reflux for 30 minutes before being cooled to 21°C over approximately 90 minutes. The white solid was collected by filtration, washed with ethanol (10 mL) and dried under vacuum at 21°C for 16 hours to afford 5-[4-[2-(N-methyl-N-(2-pyridyl)amino)ethoxy]benzyl]thiazolidine-2,4-dione γ-cyclodextrin complex.

¹H-NMR (d⁶-DMSO): consistent with approximately 1:1 5-[4-[2-(N-methyl-N-(2-pyridyl)amino)ethoxy]benzyl]thiazolidine-2,4-dione γ-cyclodextrin complex.

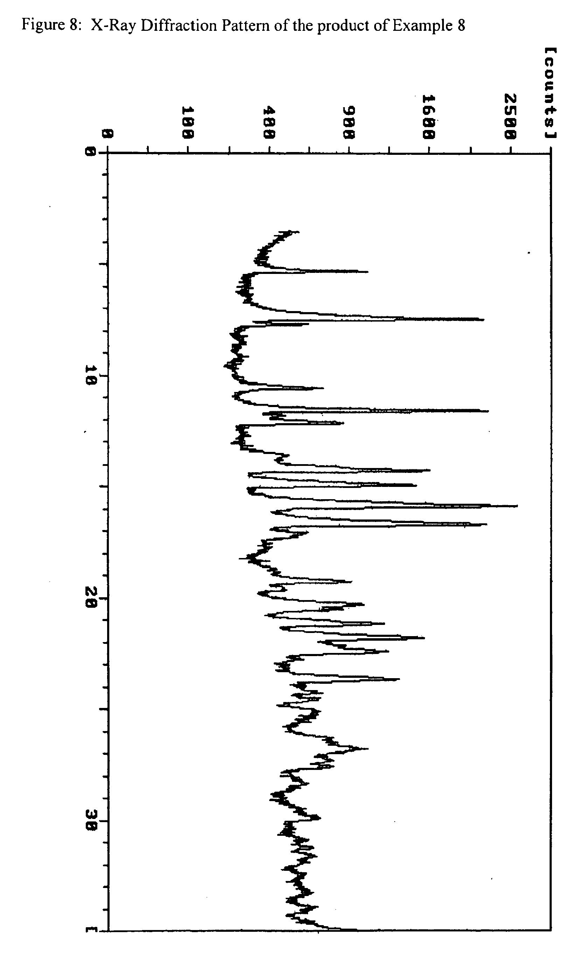

XRPD: See Figure 8. XRPD is consistent with that of the product of Example 7 (Figure 7).

### Example 9: Preparation of a 5-[4-[2-(N-methyl-N-(2-pyridyl)amino)ethoxy]benzyl] thiazolidine-2,4-dione hydroxypropyl-β-cyclodextrin complex

A solution of hydroxypropyl-β-cyclodextrin (Molecular Substitution (MS) ~0.8) (2.0 g) in water (20 ml) was heated to reflux and 5-[4-[2-(N-methyl-N-(2-pyridyl)amino)ethoxy]benzyl]thiazolidine-2,4-dione (0.49 g) was added in portions. Glacial acetic acid (1.0 g) was added and the mixture was stirred at reflux to give a clear solution. The mixture was stirred at reflux for 2-3 hours, then the solvent was removed by evaporation at 70°C *in vacuo* to afford an approximately 1:1 5-[4-[2-(N-methyl-N-(2-pyridyl)amino)ethoxy]benzyl] thiazolidine-2,4-dione hydroxypropyl-β-cyclodextrin complex as a white solid.

### Example 10: Preparation of a 5-[4-[2-(N-methyl-N-(2-pyridyl)amino)ethoxy]benzyl] thiazolidine-2,4-dione potassium salt β-cyclodextrin complex

Water (1.5 mL) was added to β-cyclodextrin (3.0 g) in a mortar and the mixture was ground until a paste was formed. 5-[4-[2-(N-Methyl-N-(2-pyridyl)amino)ethoxy]benzyl] thiazolidine-2,4-dione potassium salt (1.05 g) was added, together with another portion of water (1.5 mL) and the mixture was ground for a further 10 minutes. The paste was exposed to an open atmosphere at 21 °C for 20 hours to afford an approximately 1:1 1 5-[4-[2-(N-methyl-N-(2-pyridyl)amino)ethoxy]benzyl]thiazolidine-2,4-dione potassium salt β-cyclodextrin complex.

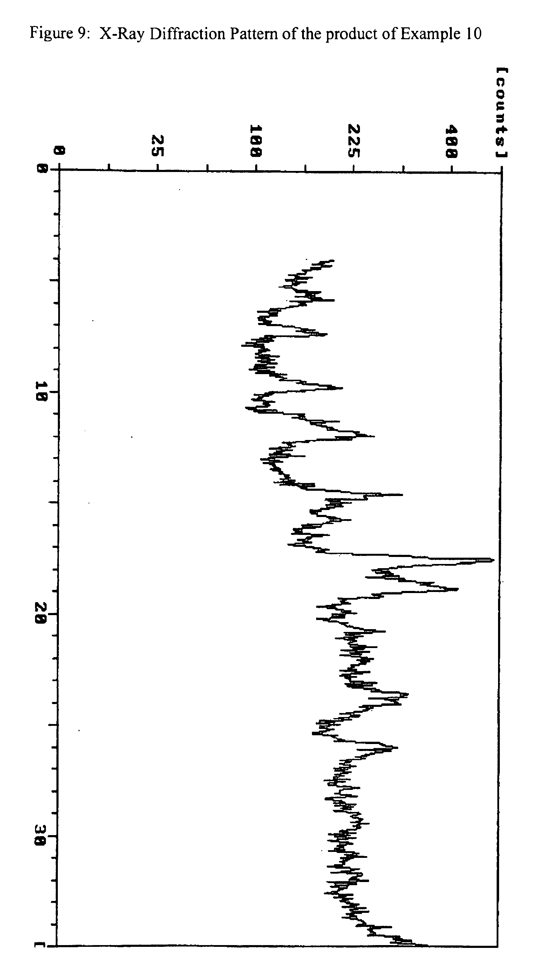

XRPD: See Figure 9.

### Example 11: Preparation of a 5-[4-[2-(N-methyl-N-(2-pyridyl)amino)ethoxy]benzyl] thiazolidine-2,4-dione potassium salt β-cyclodextrin complex

β-Cyclodextrin (6.0 g) and water (3.0 mL) were ground together in a mortar until a paste was formed. 5-[4-[2-(N-Methyl-N-(2-pyridyl)amino)ethoxy]benzyl]thiazolidine-2,4-dione potassium salt (1.05 g) and water (3.0 mL) were added and the mixture was ground for a further 15 minutes. The paste was dried at 45°C under vacuum for 3 hours to produce a 1:2 5-[4-[2-(N-methyl-N-(2-pyridyl)amino)ethoxy]benzyl]thiazolidine-2,4-dione potassium salt β-cyclodextrin complex.

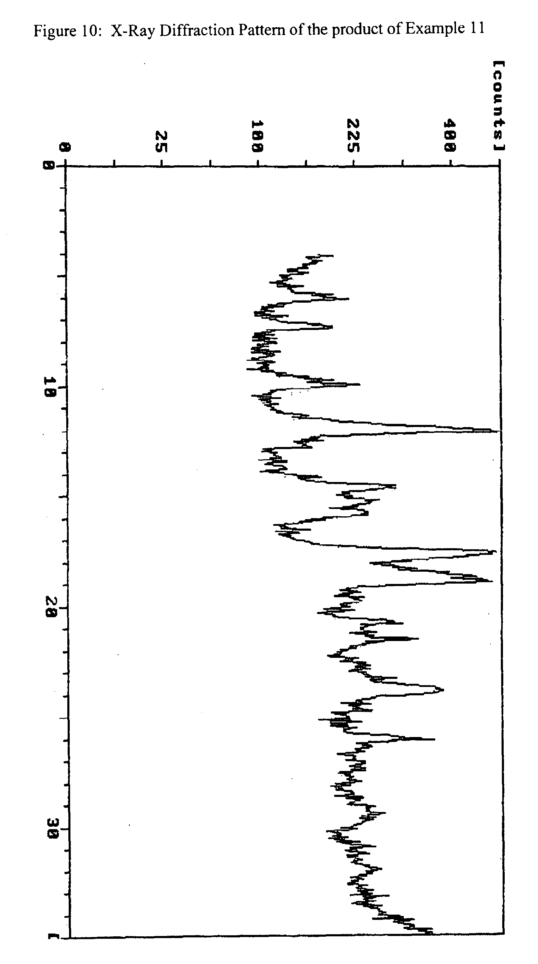

XRPD: See Figure 10.

### Example 12: Preparation of a 5-[4-[2-(N-methyl-N-(2-pyridyl)amino)ethoxy]benzyl] thiazolidine-2,4-dione potassium salt β-cyclodextrin complex

A sample of the product of Example 11 (3.0 g) was dissolved in the minimum volume of water at reflux, the solution was filtered and allowed to cool to 21°C. The solid was collected by filtration to afford 1:2 5-[4-[2-(N-methyl-N-(2-pyridyl)amino)ethoxy]benzyl]thiazolidine-2,4-dione potassium salt β-cyclodextrin complex.

¹H-NMR (d⁶-DMSO): consistent with approximately 1:2 5-[4-[2-(N-methyl-N-(2-pyridyl)amino)ethoxy]benzyl]thiazolidine-2,4-dione potassium salt β-cyclodextrin complex.

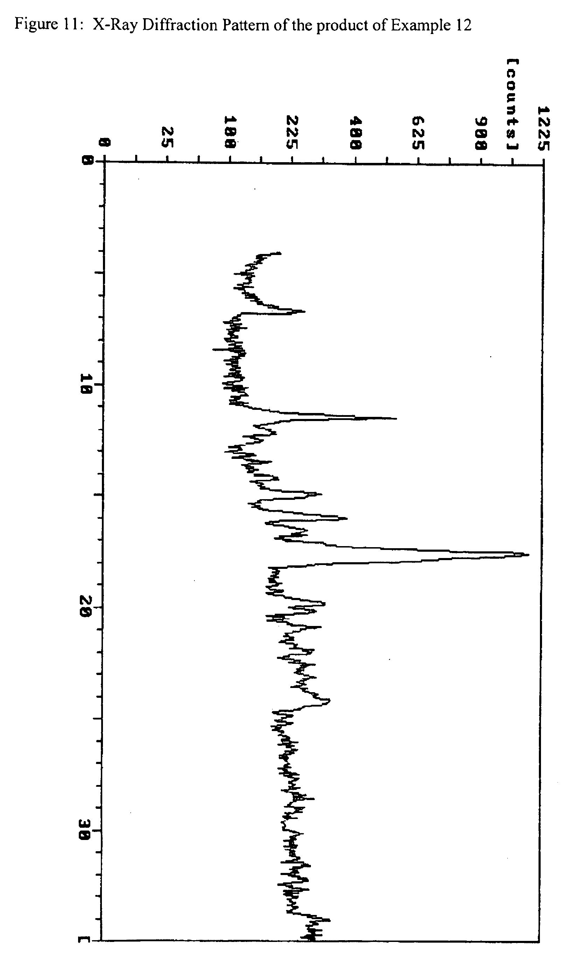

XRPD: See Figure 11.

### Example 13: Preparation of a 5-[4-[2-(N-methyl-N-(2-pyridyl)amino)ethoxy]benzyl] thiazolidine-2,4-dione potassium salt β-cyclodextrin complex

A stirred mixture of 5-[4-[2-(N-methyl-N-(2-pyridyl)amino)ethoxy]benzyl]thiazolidine-2,4-dione potassium salt (1.05 g), β-cyclodextrin (6.0 g) and approximately 10 mg of potassium hydroxide in water was heated to reflux where a clear solution was formed. The solution was cooled to 21°C over approximately 1 hour, before the precipitate was collected by filtration. The solid was dried under vacuum for 5 hours at 40°C to give 1:2 5-[4-[2-(N-methyl-N-(2-pyridyl)amino)ethoxy]benzyl]thiazolidine-2,4-dione potassium salt β-cyclodextrin complex as a white solid.

¹H-NMR (d⁶-DMSO): consistent with approximately 1:2 5-[4-[2-(N-methyl-N-(2-pyridyl)amino)ethoxy]benzyl]thiazolidine-2,4-dione potassium salt β-cyclodextrin complex.

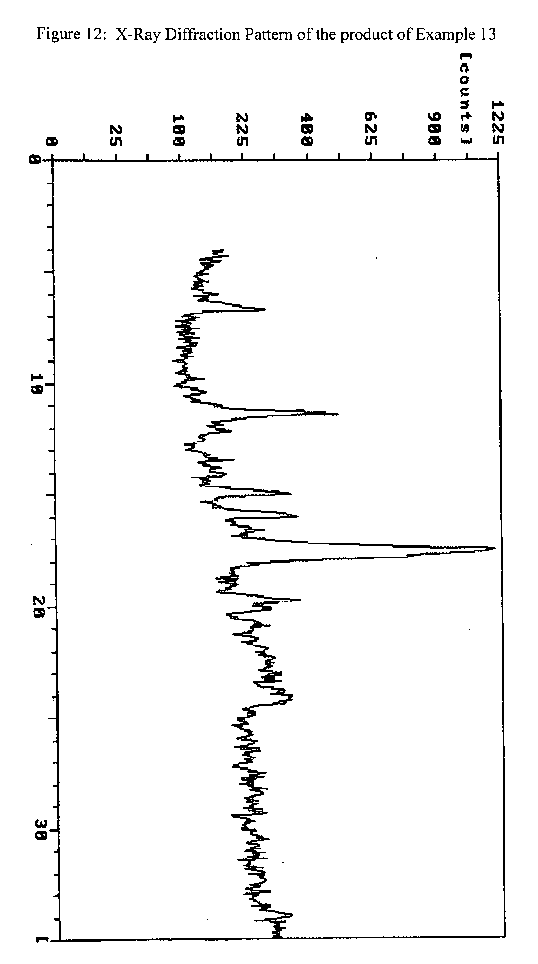

XRPD: See Figure 12. XRPD is consistent with that of the product of Example 12 (Figure 11).

### Example 14: Preparation of a 5-[4-[2-(N-methyl-N-(2-pyridyl)amino)ethoxy]benzyl] thiazolidine-2,4-dione potassium salt γ-cyclodextrin complex

5-[4-[2-(N-Methyl-N-(2-pyridyl)amino)ethoxy]benzyl]thiazolidine-2,4-dione potassium salt (0.62g) was added in portions to a solution of γ-cyclodextrin hydrate (4g) in water (60 ml) at 21 °C and the mixture was stirred to give a clear solution. Diethyl ether (30 ml) was added and the mixture was stirred vigorously. A solid precipitate was observed to form rapidly and the resulting suspension was stirred at 21 °C for a further 1 hour. The solid was filtered and dried at 21 °C under vacuum for 16 hours to afford 1:2 5-[4-[2-(N-methyl-N-(2-pyridyl)amino)ethoxy]benzyl]thiazolidine-2,4-dione potassium salt γ-cyclodextrin complex.

NMR consistent with the formation of an approximately 1:2 5-[4-[2-(N-methyl-N-(2-pyridyl)amino)ethoxy]benzyl]thiazolidine-2,4-dione potassium salt γ-cyclodextrin complex.

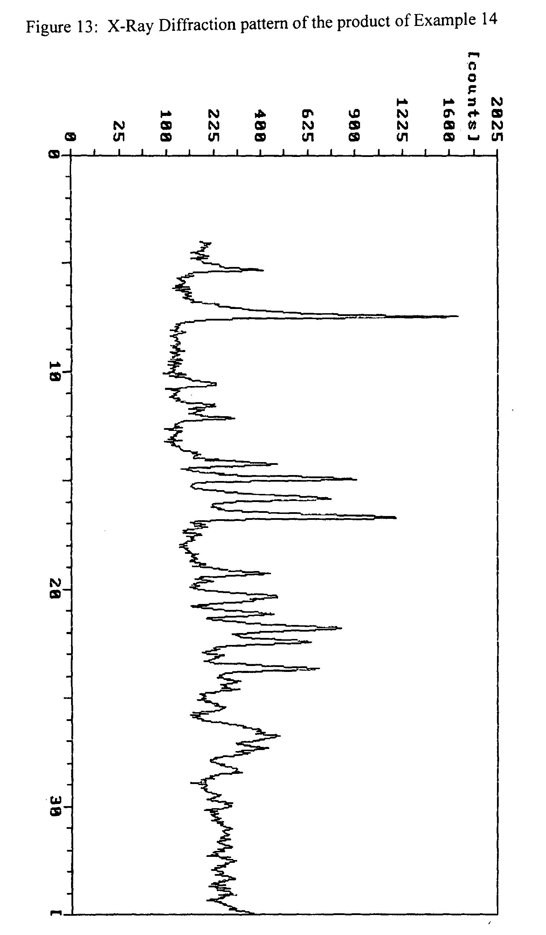

XRPD: See Figure 13.

### Example 15: Preparation of a 5-[4-[2-(N-methyl-N-(2-pyridyl)amino)ethoxy]benzyl] thiazolidine-2,4-dione potassium salt γ-cyclodextrin complex

A 1 g sample of the product from example 14 was recrystallised from water (15 ml) to afford a ~1:2 5-[4-[2-(N-methyl-N-(2-pyridyl)amino)ethoxy]benzyl]thiazolidine-2,4-dione potassium salt γ-cyclodextrin complex.

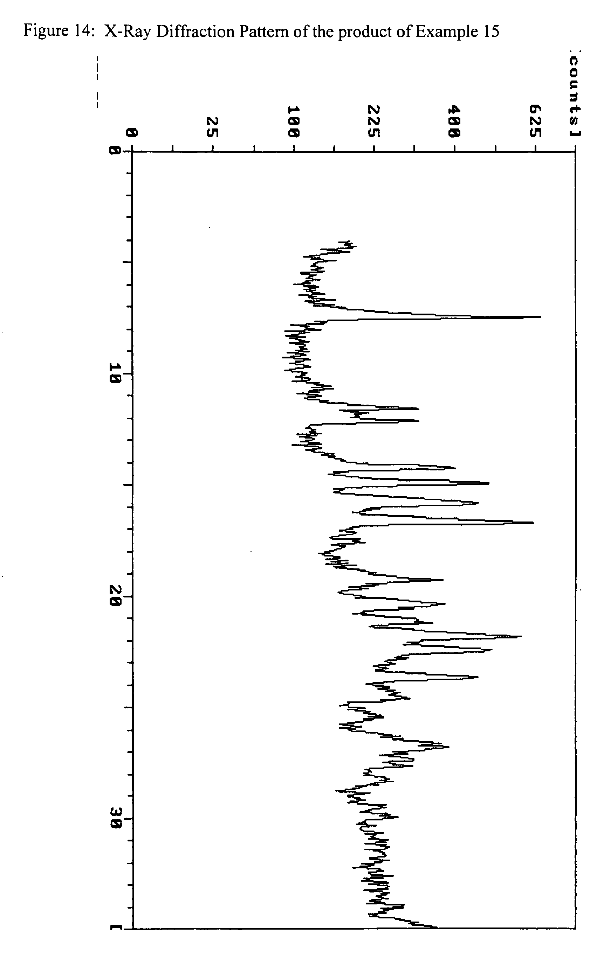

XRPD: See Figure 14.

### Example 16: Preparation of a 5-[4-[2-(N-methyl-N-(2-pyridyl)amino)ethoxy]benzyl] thiazolidine-2,4-dione potassium salt hydroxypropyl-β-cyclodextrin complex.

5-[4-[2-(N-Methyl-N-(2-pyridyl)amino)ethoxy]benzyl]thiazolidine-2,4-dione potassium salt (0.52 g) was added to a solution of hydroxypropyl-β-cyclodextrin (MS ~0.8) (2g) in water (20 ml) and the mixture was stirred to give a clear solution. The water was removed by evaporation at 70°C *in vacuo* to afford a 5-[4-[2-(N-methyl-N-(2-pyridyl)amino)ethoxy]benzyl]thiazolidine-2,4-dione potassium salt hydroxypropyl-β-cyclodextrin complex

### Example 17: Preparation of a 5-[4-[2-(N-methyl-N-(2-pyridyl)amino)ethoxy]benzyl] thiazolidine-2,4-dione hydrochloride β-cyclodextrin complex

β-Cyclodextrin (3.0 g) and water (1.5 mL) were ground in a mortar until a paste was formed. 5-[4-[2-(N-Methyl-N-(2-pyridyl)amino)ethoxy]benzyl]thiazolidine-2,4-dione hydrochloride dihydrate (1.14 g) and water (1.5 mL) were then added and the mixture was ground for 20 minutes. The paste was allowed to dry at 21 °C for 16 hours to give 5-[4-[2-(N-methyl-N-(2-pyridyl)amino)ethoxy]benzyl]thiazolidine-2,4-dione hydrochloride β-cyclodextrin complex (3.78 g).

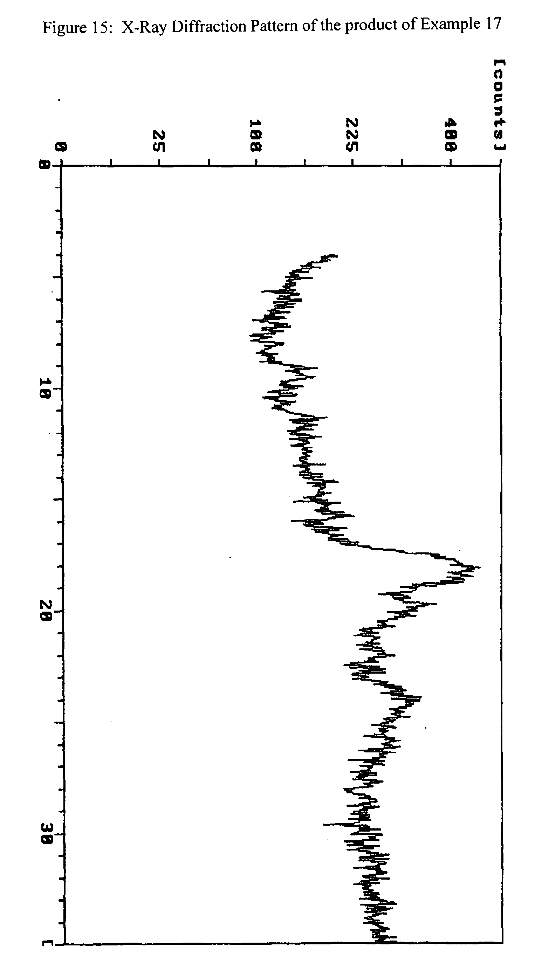

XRPD: See Figure 15.

### Example 18: Preparation of a 5-[4-[2-(N-methyl-N-(2-pyridyl)amino)ethoxy]benzyl] thiazolidine-2,4-dione hydrochloride hydroxypropyl-β-cyclodextrin complex

Hydroxypropyl-β-cyclodextrin (1.0 g) was added to a stirred solution of 5-[4-[2-(N-methyl-N-(2-pyridyl)amino)ethoxy]benzyl]thiazolidine-2,4-dione hydrochloride dihydrate (0.36 g) in water (10 mL) at 75°C. The cyclodextrin was washed in with a further 5 mL of water. After approximately one minute the stirred solution was observed to be clear and so the solution was cooled to 21 °C over approximately 60 minutes. The solvent was removed by freeze drying to afford 5-[4-[2-(N-methyl-N-(2-pyridyl)amino)ethoxy]benzyl]thiazolidine-2,4-dione hydrochloride hydroxypropyl-β-cyclodextrin complex as a white solid.

¹H-NMR (D₂O): consistent with approximately 1:1 5-[4-[2-(N-methyl-N-(2-pyridyl)amino)ethoxy]benzyl]thiazolidine-2,4-dione hydrochloride hydroxypropyl-β-cyclodextrin complex.

### Example 19: Preparation of a 5-[4-[2-(N-methyl-N-(2-pyridyl)amino)ethoxy]benzyl] thiazolidine-2,4-dione hydrochloride γ-cyclodextrin complex

5-[4-[2-(N-Methyl-N-(2-pyridyl)amino)ethoxy]benzyl]thiazolidine-2,4-dione hydrochloride (0.6g) was added to a refluxing mixture of γ-cyclodextrin hydrate (4.0g), ethanol (20 ml) and water (20 ml). The mixture was stirred at reflux for a further 2 hours, allowed to cool to 21°C and then maintained at this temperature for approximately 18 hours. The solvent was removed by evaporation at 50°C *in vacuo* to afford a 1:2 5-[4-[2-(N-Methyl-N-(2-pyridyl)amino)ethoxy]benzyl]thiazolidine-2,4-dione hydrochloride y-cyclodextrin complex as a white solid.

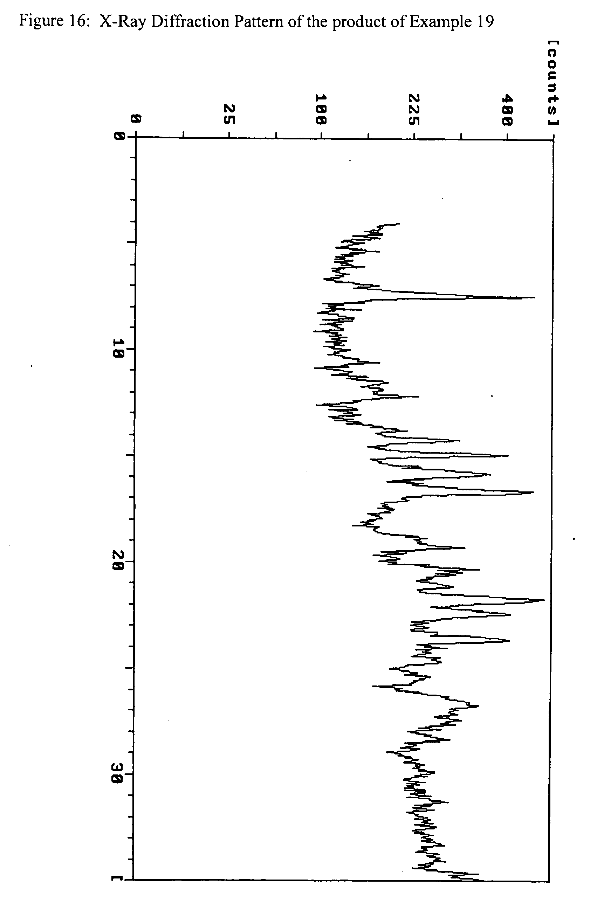

XRPD: See Figure 16.

### Example 20: Preparation of a 5-[4-[2-(N-methyl-N-(2-pyridyl)amino)ethoxy]benzyl] thiazolidine-2,4-dione maleate β-cyclodextrin complex

β-Cyclodextrin (3.0 g) and water (1.5 mL) were ground into a paste in a mortar. 5-[4-[2-(N-Methyl-N-(2-pyridyl)amino)ethoxy]benzyl]thiazolidine-2,4-dione maleate (1.25 g) and water (1.25 mL) were added and the mixture was ground for 15 minutes. The paste was allowed to dry in an open atmosphere at 21°C for 4 hours to afford 1:1 5-[4-[2-(N-methyl-N-(2-pyridyl)amino)ethoxy]benzyl]thiazolidine-2,4-dione maleate β-cyclodextrin complex as a white solid.

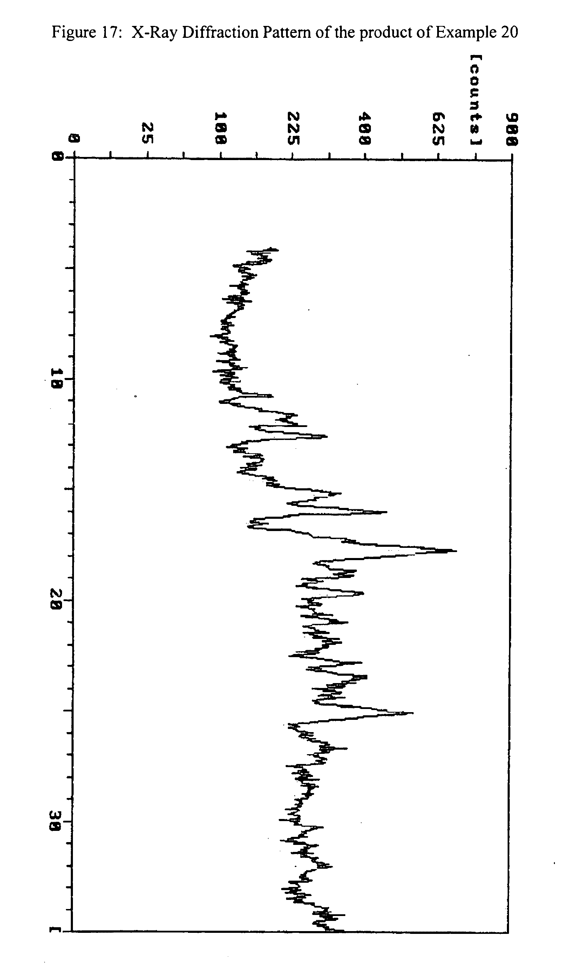

XRPD: See Figure 17.

### Example 21: Preparation of a 5-[4-[2-(N-methyl-N-(2-pyridyl)amino)ethoxy]benzyl] thiazolidine-2,4-dione maleate β-cyclodextrin complex

β-Cyclodextrin (6.0 g) and water (3.0 mL) were ground into a paste in a mortar. 5-[4-[2-(N-Methyl-N-(2-pyridyl)amino)ethoxy] benzyl]thiazolidine-2,4-dione maleate (1.25 g) and water (3.0 mL) were added and the mixture was ground together for 15 minutes. The mortar was then placed in an oven at 45°C under vacuum for 3 hours. The solid was recovered from the mortar to afford 1:2 5-[4-[2-(N-methyl-N-(2-pyridyl)amino)ethoxy]benzyl]thiazolidine-2,4-dione maleate β-cyclodextrin complex as a white solid.

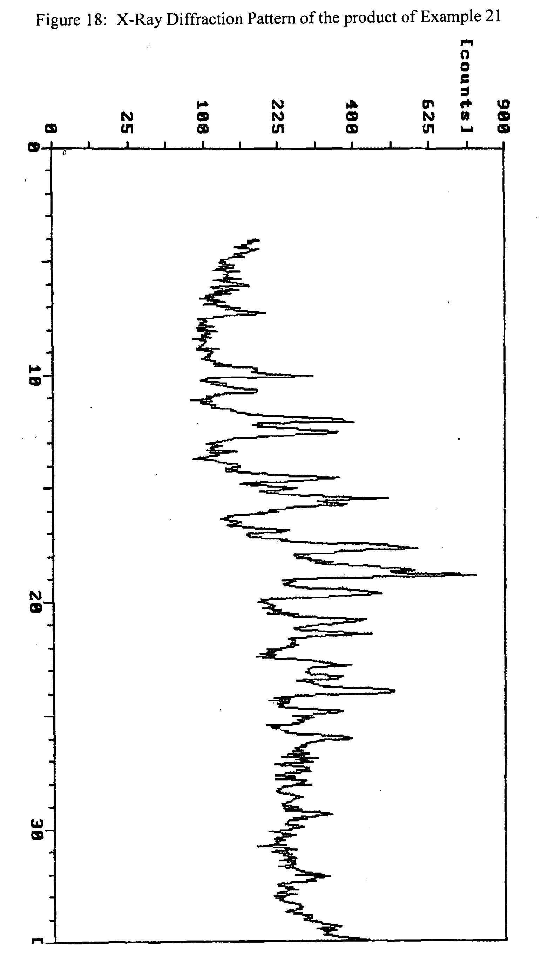

XRPD: See Figure 18.

### Example 22: Preparation of a 5-[4-[2-(N-methyl-N-(2-pyridyl)amino)ethoxy] benzyl]thiazolidine-2,4-dione maleate γ-cyclodextrin complex.

5-[4-[2-(N-methyl-N-(2-pyridyl)amino)ethoxy]benzyl]thiazolidine-2,4-dione (8.25 g) and maleic acid (2.8 g) were added to a solution of γ-cyclodextrin (30 g) in water (150 mL) at approximately 70°C. The mixture was maintained at this temperature for approximately 1 hour at which time a clear solution was observed. The solution was cooled to 21 °C and stirred at this temperature for 3 hours before being left to stand for 16 hours. The solid was collected by filtration and dried over phosphorus pentoxide under vacuum to give 5-[4-[2-(N-methyl-N-(2-pyridyl)amino)ethoxy]benzyl]thiazolidine-2,4-dione γ-cyclodextrin complex (23.1 g) as a white solid.

¹H-NMR (DMSO/D₂O): consistent with approximately 1:1.5 5-[4-[2-(N-methyl-N-(2-pyridyl)amino)ethoxy]benzyl]thiazolidine-2,4-dione maleate γ-cyclodextrin complex.

Water Content (Karl Fischer): 9.6% w/w.

HPLC Assay: consistent with the complex containing 13.7% w/w of 5-[4-[2-(N-methyl-N-(2-pyridyl)amino)ethoxy]benzyl]thiazolidine-2,4-dione.

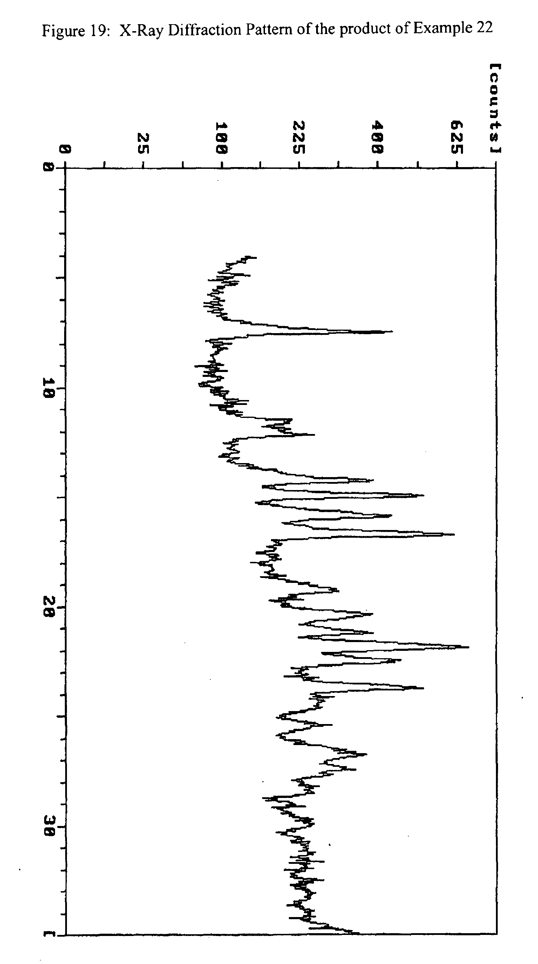

XRPD: See Figure 19.

### Example 23: Preparation of a 5-[4-[2-(N-methyl-N-(2-pyridyl)amino)ethoxy] benzyl]thiazolidine-2,4-dione maleate hydroxypropyl-β-cyclodextrin complex.

5-[4-[2-(N-Methyl-N-(2-pyridyl)amino)ethoxy]benzyl]thiazolidine-2,4-dione maleate (6.48 g) was added to a solution of hydroxypropyl-β-cyclodextrin (MS ~ 0.8, 20.0 g) in water (100 mL) at 70°C. The mixture was maintained at approximately 70°C for 1 hour before the solvent was removed from the clear solution by evaporation under reduced pressure at 50°C to afford a 5-[4-[2-(N-methyl-N-(2-pyridyl)amino)ethoxy]benzyl] thiazolidine-2,4-dione maleate hydroxypropyl-β-cyclodextrin complex (19.9 g).

¹H-NMR (DMSO/D₂O): consistent with approximately 1:0.9 5-[4-[2-(N-methyl-N-(2-pyridyl)amino)ethoxy]benzyl]thiazolidine-2,4-dione maleate hydroxypropyl-β-cyclodextrin complex.

Water Content (Karl Fischer): 5.9% w/w.

HPLC Assay: consistent with the complex containing 18.2% w/w of 5-[4-[2-(N-methyl-N-(2-pyridyl)amino)ethoxy]benzyl]thiazolidine-2,4-dione.

### Example 24: Preparation of a 5-[4-[2-(N-methyl-N-(2-pyridyl)amino)ethoxy] benzyl]thiazolidine-2,4-dione maleate hydroxypropyl-γ-cyclodextrin complex.

5-[4-[2-(N-Methyl-N-(2-pyridyl)amino)ethoxy]benzyl]thiazolidine-2,4-dione maleate (3.6 g) was added to a solution of hydroxypropyl-γ-cyclodextrin (MS ~ 0.6, 12.0 g) in water (75 mL) at 70°C. The clear solution was stirred at 70°C for 2 hours then the solvent was removed under vacuum at approximately 50°C to give a 5-[4-[2-(N-methyl-N-(2-pyridyl)amino)ethoxy]benzyl]thiazolidine-2,4-dione maleate hydroxypropyl-γ-cyclodextrin complex (13.4 g).

¹H-NMR (DMSO/D₂O): consistent with approximately 1:1 5-[4-[2-(N-methyl-N-(2-pyridyl)amino)ethoxy]benzyl]thiazolidine-2,4-dione maleate hydroxypropyl-γ-cyclodextrin complex.

Water Content (Karl Fischer): 5.6% w/w.

HPLC Assay: consistent with the complex containing 16.9% w/w of 5-[4-[2-(N-methyl-N-(2-pyridyl)amino)ethoxy]benzyl]thiazolidine-2,4-dione.

### Example 25: Preparation of a 5-[4-[2-(N-methyl-N-(2-pyridyl)amino)ethoxy]benzyl]thiazolidine-2,4-dione γ-cyclodextrin complex.

5-[4-[2-(N-Methyl-N-(2-pyridyl)amino)ethoxy]benzyl]thiazolidine-2,4-dione hydrochloride dihydrate (6.1 g) was added to a solution of γ-cyclodextrin (20 g) in water (200 mL) at 21 °C. Aqueous potassium carbonate solution (22 mL, 10% w/v) was added dropwise to the clear solution, resulting in a solid precipitate. The suspension was stirred for approximately 2 hours at 21 °C before the solid was collected by filtration and dried over phosphorus pentoxide to afford 5-[4-[2-(N-methyl-N-(2-pyridyl)amino)ethoxy]benzyl]thiazolidine-2,4-dione γ-cyclodextrin complex (21.5 g).

¹H-NMR (DMSO): consistent with approximately 1:0.9 5-[4-[2-(N-methyl-N-(2-pyridyl)amino)ethoxy]benzyl]thiazolidine-2,4-dione γ-cyclodextrin complex.

Water Content (Karl Fischer): 10.0% w/w.

HPLC Assay: consistent with the complex containing 20.5% w/w of 5-[4-[2-(N-methyl-N-(2-pyridyl)amino)ethoxy]benzyl]thiazolidine-2,4-dione.

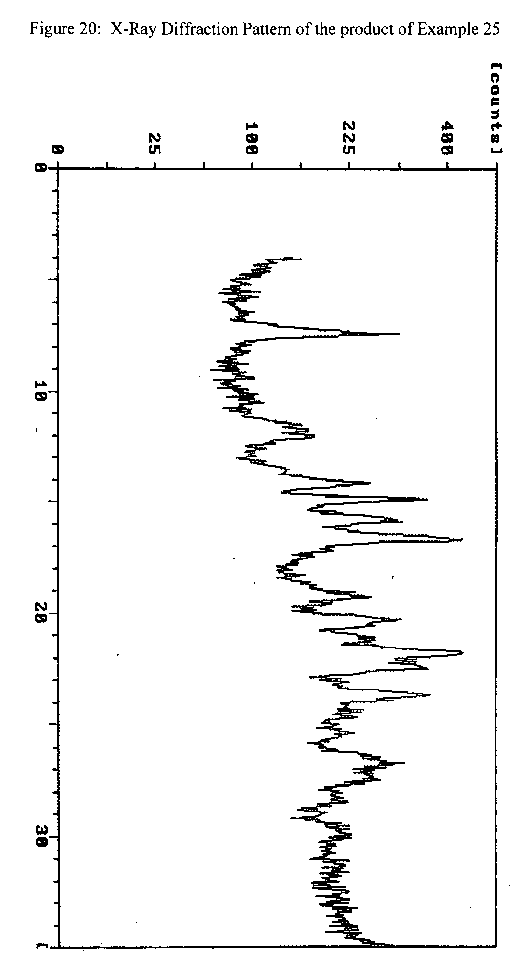

XRPD: See Figure 20.

### Example 26: Preparation of a 5-[4-[2-(N-methyl-N-(2-pyridyl)amino)ethoxy]benzyl]thiazolidine-2,4-dione hydroxypropyl-β-cyclodextrin complex.

A solution of hydroxypropyl-β-cyclodextrin (MS ~0.8) (2g) in water (20 ml) was heated to 75°C. A solution of 5-[4-[2-(N-methyl-N-(2-pyridyl)amino)ethoxy]benzyl]thiazolidine-2,4-dione (0.27 g) in tetrahydrofuran (10 ml) was added dropwise. The mixture was stirred at reflux for 2-3 hours, then the solution was filtered hot and the solvent was removed from the filtrate by evaporation at 70°C *in vacuo* to afford 5-[4-[2-(N-methyl-N-(2-pyridyl)amino)ethoxy]benzyl]thiazolidine-2,4-dione hydroxypropyl-β-cyclodextrin complex as a white solid.

### Example 27: Preparation of a 5-[4-[2-(N-methyl-N-(2-pyridyl)amino)ethoxy] benzyl]thiazolidine-2,4-dione hydroxypropyl-β-cyclodextrin complex.

A solution of 5-[4-[2-(N-methyl-N-(2-pyridyl)amino)ethoxy]benzyl]thiazolidine-2,4-dione (2.5 g) in tetrahydrofuran (50 ml) was added dropwise to a solution of hydroxypropyl-β-cyclodextrin (MS ~0.8) (20 g) in water (75 ml) and THF (25 mL) at reflux. The solution was stirred at reflux for 2 hours, before the solvent was removed by evaporation at 50°C *in vacuo* to afford 5-[4-[2-(N-methyl-N-(2-pyridyl)amino)ethoxy] benzyl]thiazolidine-2,4-dione hydroxypropyl-β-cyclodextrin complex (18.4 g).

¹H-NMR (DMSO/D₂O): consistent with approximately 1:2 5-[4-[2-(N-methyl-N-(2-pyridyl)amino)ethoxy]benzyl]thiazolidine-2,4-dione hydroxypropyl-β-cyclodextrin complex.

Water Content (Karl Fischer): 7.1% w/w.

### Example 28: Preparation of a 5-[4-[2-(N-methyl-N-(2-pyridyl)amino)ethoxy] benzyl]thiazolidine-2,4-dione hydroxypropyl-γ-cyclodextrin complex.

A solution of 5-[4-[2-(N-methyl-N-(2-pyridyl)amino)ethoxy]benzyl]thiazolidine-2,4-dione (1.8 g) in tetrahydrofuran (45 ml) was added dropwise to a solution of hydroxypropyl-γ-cyclodextrin (15 g) in water (75 mL) at approximately 70°C. The reaction was maintained at this temperature for 15 minutes. The solvent was then removed under reduced pressure at 70°C to afford 5-[4-[2-(N-methyl-N-(2-pyridyl)amino)ethoxy]benzyl]thiazolidine-2,4-dione hydroxypropyl-γ-cyclodextrin complex as a white solid.

¹H-NMR (DMSO/D₂O): consistent with approximately 1:1.5 5-[4-[2-(N-methyl-N-(2-pyridyl)amino)ethoxy]benzyl]thiazolidine-2,4-dione hydroxypropyl-γ-cyclodextrin complex.
Water Content (Karl Fischer): 10.7% w/w.

### Example 29: Preparation of a 5-[4-[2-(N-methyl-N-(2-pyridyl)amino)ethoxy] benzyl]thiazolidine-2,4-dione potassium salt γ-cyclodextrin complex.

5-[4-[2-(N-Methyl-N-(2-pyridyl)amino)ethoxy]benzyl]thiazolidine-2,4-dione potassium salt (4.34 g) was added to a solution of y-cyclodextrin (28 g) in water (400 mL) at 21 °C. The solution was filtered and diethyl ether (200 mL) was added to the filtrate, with vigorous stirring. The suspension was stirred at 21 °C for 1 hour then the solid was collected by filtration and dried over phosphorus pentoxide under vacuum for 16 hours to afford 5-[4-[2-(N-methyl-N-(2-pyridyl)amino)ethoxy]benzyl]thiazolidine-2,4-dione potassium salt γ-cyclodextrin complex (17.6 g).

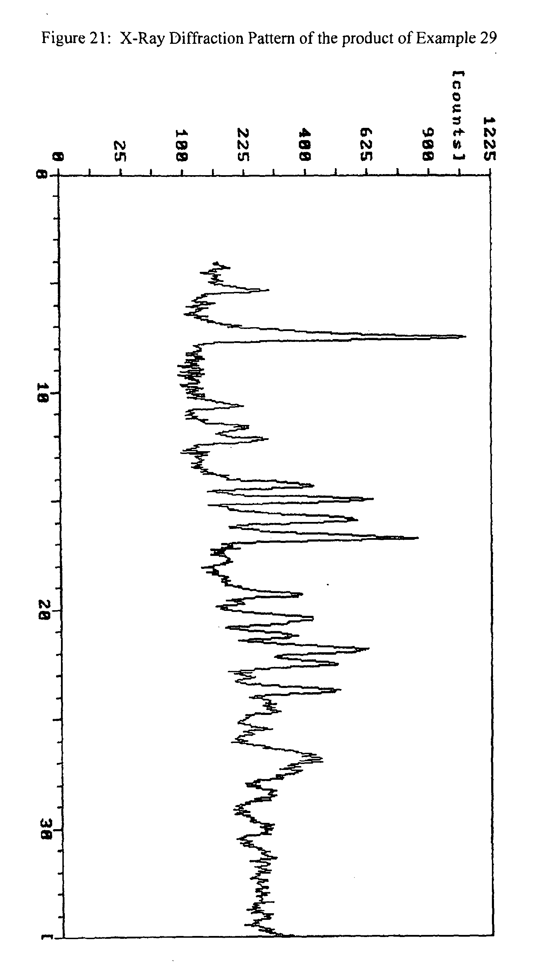

XRPD: See Figure 21.

### Example 30: Preparation of a 5-[4-[2-(N-methyl-N-(2-pyridyl)amino)ethoxy] benzyl]thiazolidine-2,4-dione potassium salt γ-cyclodextrin complex.

The product of Example 29 (15 g) was recrystallised from water (50 mL) and dried for 16 hours to give 5-[4-[2-(N-methyl-N-(2-pyridyl)amino)ethoxy] benzyl]thiazolidine-2,4-dione potassium salt γ-cyclodextrin complex.

¹H-NMR (D₂O): consistent with approximately **1:2** 5-[4-[2-(N-methyl-N-(2-pyridyl)amino)ethoxy]benzyl]thiazolidine-2,4-dione potassium salt γ-cyclodextrin complex.

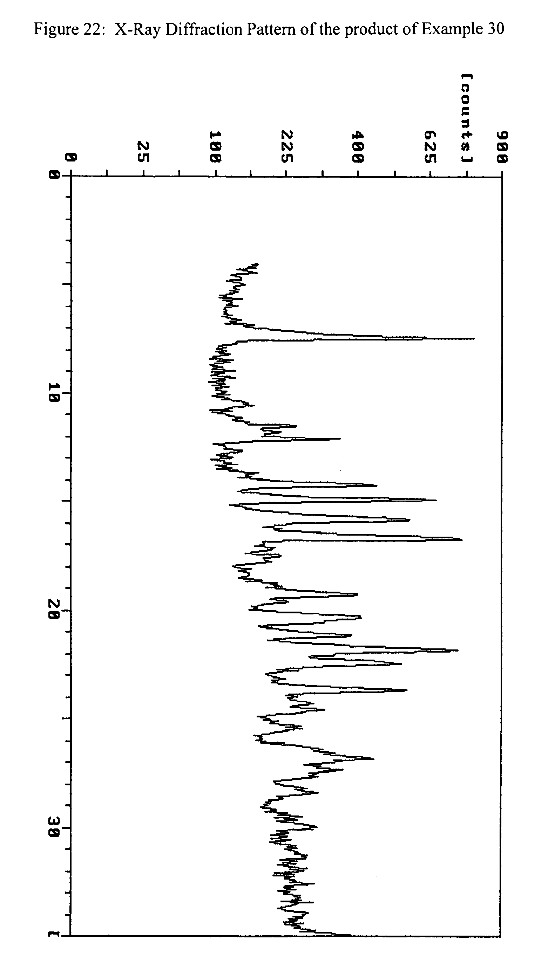

XRPD: See Figure 22.

### Example 31: Preparation of a 5-[4-[2-(N-methyl-N-(2-pyridyl)amino)ethoxy] benzyl]thiazolidine-2,4-dione potassium salt hydroxypropyl-β-cyclodextrin complex.

5-[4-[2-(N-Methyl-N-(2-pyridyl)amino)ethoxy]benzyl]thiazolidine-2,4-dione potassium salt (3.25 g) was added to a solution of hydroxypropyl-β-cyclodextrin (MS ~ 0.8, 12 g) in water (120 mL) at 21 °C. The solvent was removed under reduced pressure at 70°C to give 5-[4-[2-(N-methyl-N-(2-pyridyl)amino)ethoxy]benzyl]thiazolidine-2,4-dione potassium salt hydroxypropyl-β-cyclodextrin complex (13.4 g) as a white solid.

### Example 32: Preparation of a 5-[4-[2-(N-methyl-N-(2-pyridyl)amino)ethoxy] benzyl]thiazolidine-2,4-dione hydrochloride γ-cyclodextrin complex.

A mixture of γ-cyclodextrin (20 g), ethanol (100 mL) and water (100 mL) was heated to reflux before 5-[4-[2-(N-methyl-N-(2-pyridyl)amino)ethoxy]benzyl]thiazolidine-2,4-dione hydrochloride dihydrate (3.3 g) was added. The clear solution was maintained at reflux for 30 minutes then the solvent was removed at 50°C under reduced pressure to afford 5-[4-[2-(N-methyl-N-(2-pyridyl)amino)ethoxy] benzyl]thiazolidine-2,4-dione hydrochloride γ-cyclodextrin complex (19 g).

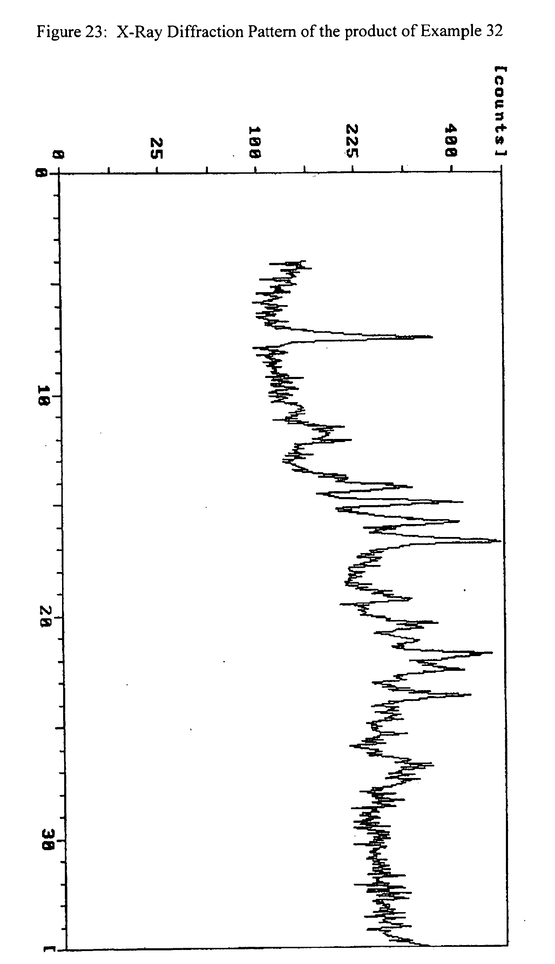

XRPD: See Figure 23.

### Example 33: Preparation of a 5-[4-[2-(N-methyl-N-(2-pyridyl)amino)ethoxy] benzyl]thiazolidine-2,4-dione hydrochloride hydroxypropyl-β-cyclodextrin complex.

5-[4-[2-(N-Methyl-N-(2-pyridyl)amino)ethoxy]benzyl]thiazolidine-2,4-dione hydrochloride dihydrate (2.3 g) was added to a solution of hydroxypropyl-b-cyclodextrin (MS ~ 0.6, 15 g) in water (150 mL) at 21°C. The solvent was removed from the clear solution at 70°C under reduced pressure to give 5-[4-[2-(N-methyl-N-(2-pyridyl)amino)ethoxy] benzyl]thiazolidine-2,4-dione hydrochloride hydroxypropyl-β-cyclodextrin complex (15.7 g) as a white solid.

### Data: Intrinsic Dissolution Rate (IDR) at pH 6.8 of the cyclodextrin complexes

The intrinsic dissolution rate was measured with a Sotax intrinsic dissolution assembly according to the procedure described in USP<1087>. The 8mm diameter disc was formed from approximately 150mg of cyclodextrin complex and compressed at a force of 5 tons for 30 seconds using a benchtop hydraulic press. The dissolution media was 1000 mL of water containing 31.6 mL of 1M hydrochloric acid solution and 2.0 g of sodium chloride, which was adjusted to pH 6.8 with Tris / Acetate buffer, the dissolution volume was 900 mL at 37°C and the rotating speed was 100 rpm. Detection was performed by flow through UV sampling every 2 minutes for 1 hour at 245 nm with a 1 cm path length.

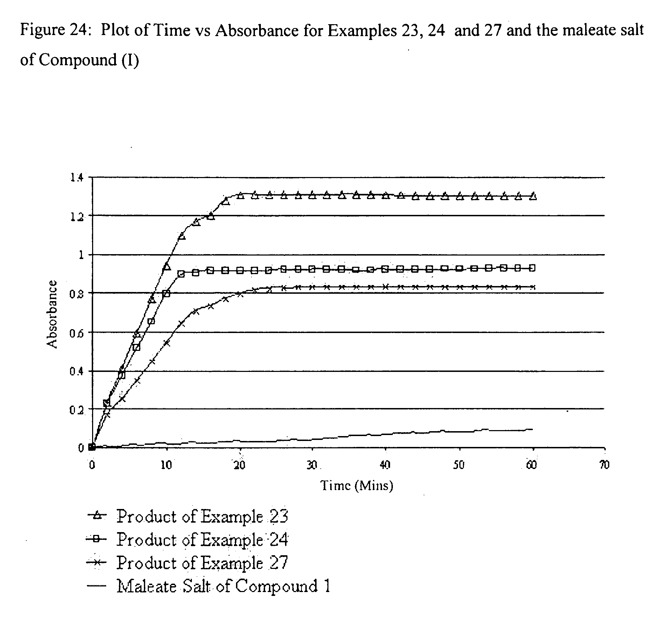

The results are presented in Figure 24 'Plot of Time vs Absorbance for Examples 23, 24 and 27 and the maleate salt of Compound (I)' and Table 1, which shows the slope of the curve, the IDR and the IDR relative to that of the Maleate salt of Compound 1.

**Table 1**

| Material | IDR (mg/min/cm²) | IDR relative to the Maleate salt of Compound 1 |
|---|---|---|
| Product of Example 23 | 3.21 | 56.7 |
| Product of Example 24 | 2.64 | 46.5 |
| Product of Example 27 | 1.90 | 33.5 |
| Maleate salt of Compound 1 | 0.057 | 1 |

## Claims

1. A host-guest complex, wherein the host is a cyclodextrin or a mixture of cyclodextrins (the Cyclodextrin) and the guest is 5-[4-[2-(N-methyl-N-(2-pyridyl)amino)ethoxy]benzyl]thiazolidine-2,4-dione, (Compound (I)) or a pharmaceutically acceptable derivative thereof or a mixture thereof.

2. A host-guest complex according to claim 1, in crystalline form.

3. A host-guest complex according to claim 1, in non-crystalline form.

4. A host-guest complex according to any one of claims 1 to 3, wherein the complex is stoichiometric or substantially stoichiometric.

5. A host-guest complex according to claim 4, wherein the stoichiometry is within the range of between about 3: 1 to about 1:1 the Cyclodextrin to Compound (I).

6. A host-guest complex according to any one of claims 1 to 5, wherein the Cyclodextrin is selected from the group consisting of α, β and γ cyclodextrins, methylated cyclodextrins, hydroxypropyl cyclodextrins e.g. hydroxypropyl-β-cyclodextrin (HPBCD) and hydroxypropyl-γ-cyclodextrin, hydroxyethyl cyclodextrins e.g. hydroxyethyl- β-cyclodextrin (HEBCD), branched cyclodextrins in which one or two glucoses or maltoses are attached to the cyclodextrin ring, ethyl- and ethyl-carboxymethyl cyclodextrins, dihydroxypropyl cyclodextrins, and sulfoalkyl ether cyclodextrins.

7. A host-guest complex according to any one of claims 1 to 6, wherein the Cyclodextrin is a α cyclodextrin.

8. A host-guest complex according to any one of claims 1 to 6, wherein the Cyclodextrin is a β cyclodextrin.

9. A host-guest complex according to any one of claims 1 to 6, wherein the Cyclodextrin is a γ cyclodextrin.

10. A host-guest complex according to any one of claims 1 to 6, wherein the Cyclodextrin is a methylated cyclodextrin.

11. A host-guest complex according to any one of claims 1 to 6, wherein the Cyclodextrin is a hydroxypropyl cyclodextrin.

12. A host-guest complex according to any one of claims 1 to 6, wherein the Cyclodextrin is a hydroxypropyl-β-cyclodextrin (HPBCD).

13. A host-guest complex according to any one of claims 1 to 6, wherein the Cyclodextrin is a hydroxypropyl-γ-cyclodextrin.

14. A host-guest complex according to any one of claims 1 to 13, wherein the guest is selected from: Compound (I), an acid salt of Compound (I) or a base salt of Compound (I).

15. A host-guest complex according to claim 1, selected from:
5-[4-[2-(N-methyl-N-(2-pyridyl)amino)ethoxy]benzyl] thiazolidine-2,4-dione β-cyclodextrin complex;
5-[4-[2-(N-methyl-N-(2-pyridyl)amino)ethoxy]benzyl] thiazolidine-2,4-dione γ-cyclodextrin complex;
5-[4-[2-(N-methyl-N-(2-pyridyl)amino)ethoxy]benzyl] thiazolidine-2,4-dione hydroxypropyl-β-cyclodextrin complex;
5-[4-[2-(N-methyl-N-(2-pyridyl)amino)ethoxy]benzyl] thiazolidine-2,4-dione potassium salt β-cyclodextrin complex;
5-[4-[2-(N-methyl-N-(2-pyridyl)amino)ethoxy]benzyl] thiazolidine-2,4-dione potassium salt γ-cyclodextrin complex;
5-[4-[2-(N-methyl-N-(2-pyridyl)amino)ethoxy]benzyl] thiazolidine-2,4-dione potassium salt hydroxypropyl-β-cyclodextrin complex;
5-[4-[2-(N-methyl-N-(2-pyridyl)amino)ethoxy]benzyl] thiazolidine-2,4-dione hydrochloride β-cyclodextrin complex;
5-[4-[2-(N-methyl-N-(2-pyridyl)amino)ethoxy]benzyl] thiazolidine-2,4-dione hydrochloride hydroxypropyl-β-cyclodextrin complex;
5-[4-[2-(N-methyl-N-(2-pyridyl)amino)ethoxy]benzyl] thiazolidine-2,4-dione hydrochloride γ-cyclodextrin complex;
5-[4-[2-(N-methyl-N-(2-pyridyl)amino)ethoxy]benzyl] thiazolidine-2,4-dione maleate β-cyclodextrin complex;
5-[4-[2-(N-methyl-N-(2-pyridyl)amino)ethoxy] benzyl]thiazolidine-2,4-dione maleate γ-cyclodextrin complex;
5-[4-[2-(N-methyl-N-(2-pyridyl)amino)ethoxy] benzyl]thiazolidine-2,4-dione maleate hydroxypropyl-β-cyclodextrin complex;
5-[4-[2-(N-methyl-N-(2-pyridyl)amino)ethoxy] benzyl]thiazolidine-2,4-dione maleate hydroxypropyl-γ-cyclodextrin complex;
5-[4-[2-(N-methyl-N-(2-pyridyl)amino)ethoxy]benzyl]thiazolidine-2,4-dione γ-cyclodextrin complex;
5-[4-[2-(N-methyl-N-(2-pyridyl)amino)ethoxy]benzyl]thiazolidine-2,4-dione hydroxypropyl-β-cyclodextrin complex;
5-[4-[2-(N-methyl-N-(2-pyridyl)amino)ethoxy] benzyl]thiazolidine-2,4-dione hydroxypropyl-β-cyclodextrin complex;
5-[4-[2-(N-methyl-N-(2-pyridyl)amino)ethoxy] benzyl]thiazolidine-2,4-dione hydroxypropyl-γ-cyclodextrin complex;
5-[4-[2-(N-methyl-N-(2-pyridyl)amino)ethoxy] benzyl]thiazolidine-2,4-dione potassium salt γ-cyclodextrin complex;
5-[4-[2-(N-methyl-N-(2-pyridyl)amino)ethoxy] benzyl]thiazolidine-2,4-dione potassium salt γ-cyclodextrin complex;.
5-[4-[2-(N-methyl-N-(2-pyridyl)amino)ethoxy] benzyl]thiazolidine-2,4-dione potassium salt hydroxypropyl-β-cyclodextrin complex;
5-[4-[2-(N-methyl-N-(2-pyridyl)amino)ethoxy] benzyl]thiazolidine-2,4-dione hydrochloride γ-cyclodextrin complex; and
5-[4-[2-(N-methyl-N-(2-pyridyl)amino)ethoxy] benzyl]thiazolidine-2,4-dione hydrochloride hydroxypropyl-β-cyclodextrin complex.

16. A process for the preparation of a host-guest complex, wherein the host is a cyclodextrin or a mixture of cyclodextrins (hereinafter also referred to as "the Cyclodextrin") and the guest is Compound (I) or a pharmaceutically acceptable derivative thereof or a mixture thereof, which process comprises contacting the Cyclodextrin and Compound (I) or a pharmaceutically acceptable derivative thereof or a mixture thereof in an appropriate liquid (solvent); and thereafter, if required, isolating the host-guest complex from the reaction mixture.

17. A process for the preparation of a host-guest complex, wherein the host is a cyclodextrin or a mixture of cyclodextrins (hereinafter also referred to as "the Cyclodextrin") and the guest is Compound (I) or a pharmaceutically acceptable derivative thereof or a mixture thereof, which process comprises
(a) admixing the Cyclodextrin and Compound (I) and/or a pharmaceutically acceptable derivative thereof with water and wherein the admixing process is continued until the complex formation is complete; or
(b) neutralising a solution or dispersion of the Cyclodextrin and a derivative of Compound (I), in an aqueous liquid, wherein the neutralisation is effected by addition of an appropriate neutralising reagent, so as to precipitate the required host-guest complex; or
(c) admixing a solution of Compound (I), and/or a pharmaceutically acceptable derivative thereof, in a first solvent, with a solution of a Cyclodextrin in a second solvent, wherein the first and second solvent are chosen such that the required host-guest composition precipitates from the solvent mixture; or
(d) admixing a solution of the Cyclodextrin and Compound (I), and/or a pharmaceutically acceptable derivative thereof in an aqueous liquid, with an appropriate anti-solvent so that the required host-guest composition precipitates from the solvent mixture; or
(e) preparing a solution of the host-guest composition by mixing Cyclodextrin and Compound (I), and/or a pharmaceutically acceptable derivative thereof, in an appropriate solvent and thereafter removing the solvent; or
(f) Generating a dispersion of Cyclodextrin and a Compound (I), and/or a pharmaceutically acceptable derivative thereof, in a liquid and stirring until complex formation is complete;
and thereafter as necessary isolating the required host-guest composition, for example by filtration.

18. A pharmaceutical composition comprising a host-guest complex, wherein the host is a Cyclodextrin and the guest is Compound (I) or a pharmaceutically acceptable derivative thereof or a mixture thereof, and a pharmaceutically acceptable carrier therefor

19. A method for the treatment and/or prophylaxis of diabetes mellitus, conditions associated with diabetes mellitus and certain complications thereof, in a human or non-human mammal which comprises administering an effective, non-toxic, amount of a host-guest complex, wherein the host is a Cyclodextrin and the guest is Compound (I) or a pharmaceutically acceptable derivative thereof or a mixture, thereof to a human or non-human mammal in need thereof.

20. The use of a host-guest complex, wherein the host is a Cyclodextrin and the guest is Compound (I) or a pharmaceutically acceptable derivative thereof or a mixture thereof, for the manufacture of a medicament for the treatment and/or prophylaxis of diabetes mellitus, conditions associated with diabetes mellitus and certain complications thereof.

21. A pharmaceutical composition comprising a host-guest complex, wherein the host is a Cyclodextrin and the guest is Compound (I) or a pharmaceutically acceptable derivative thereof or a mixture thereof in combination with one or more other pharmaceutically active agent and optionally a pharmaceutically acceptable carrier therefor.
